# EUROPEAN PATENT APPLICATION

(11) **EP 4 844 012 A1**
(43) Date of publication of application: **25.11.2026**
(21) Application number: 26754061.5
(22) Date of filing: 09.02.2026
(51) Int. Cl.: C08G 59/40, C07D 307/52, C08K 5/17, C08L 63/00, C09D 7/63, C09D 163/00

(54) **EPOXY RESIN CURING AGENT, EPOXY RESIN COMPOSITION, AND AMINE COMPOUND**

(30) Priority: 13.02.2025 JP 2025021324
(71) Applicant: MITSUBISHI GAS CHEMICAL COMPANY, INC., Chiyoda-ku Tokyo 100-8324 (JP)
(72) Inventor: IKARASHI, Yuki, Tokyo 100-8324 (JP); INNAMI, Hiroki, Tokyo 100-8324 (JP); SHIRAI, Shinyou, Tokyo 100-8324 (JP); KUMANO, Tatsuyuki, Tokyo 100-8324 (JP); ETOU, Hajime, Tokyo 100-8324 (JP)
(74) Representative: Gille Hrabal Partnerschaftsgesellschaft mbB Patentanwälte
(86) International application number: PCT/JP2026/004627
(87) International publication number: WO 2026/172936

(57) **Abstract**

Provided is an epoxy resin curing agent comprising at least one member selected from the group consisting of an amine compound (a) represented by the general formula (1) given below, and a modified product (A) of the amine compound (a). The present invention provides an epoxy resin curing agent and an epoxy resin composition which can produce a cured product excellent in toughness, impact strength, adhesion, and chemical resistance. wherein, in the formula (1), R¹ is a linear alkylene group having 1 to 3 carbon atoms, and R² and R³ are each independently a linear or branched alkylene group having 2 to 4 carbon atoms.

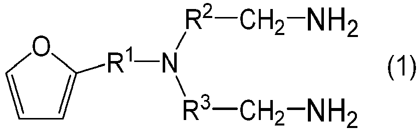

## Description

### Technical Field

The present invention relates to an epoxy resin curing agent, an epoxy resin composition, and an amine compound.

### Background Art

Various polyamine compounds are widely known as epoxy resin curing agents. In addition, compounds of polyamine modified with an epoxy compound or the like (modified polyamine) are also known to be useful as epoxy resin curing agents.

Epoxy resin compositions prepared using the polyamine compounds or the modified polyamine as epoxy resin curing agents are used not only in the field of paints such as corrosion-resistant paints for boats and ships, bridges, and onshore and offshore iron structures but also in the field of civil engineering and architecture as lining, reinforcing, and repairing materials for concrete structures, floor materials for architectural structures, linings for water supply and sewerage systems, paving materials, adhesives, and the like.

As an example in which polyamine or modified polyamine is used as an epoxy resin curing agent, for example, PTL1 discloses an amine compound represented by the general formula given below, the amine compound having a low active hydrogen equivalent weight, having sufficient curability even when used as an epoxy resin curing agent in a small formulation in an epoxy resin composition, and being able to confer favorable cured product performance. The patent literature also discloses an amine composition and an epoxy resin curing agent containing the amine compound. It should be noted that, in the formula, A is a 1,2-phenylene group, a 1,3-phenylene group, or a 1,4-phenylene group.

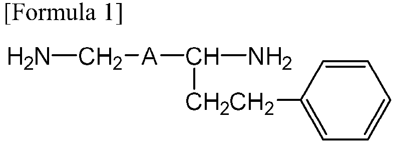

### Citation List

### Patent Literature

PTL1: WO 2017/175740

### Summary of Invention

### Technical Problem

Cured coating films of epoxy resin compositions for use under severe conditions, such as paints or linings, are required to have higher toughness, impact strength, adhesion, and chemical resistance. Furthermore, resins derived from biomass have also been developed in recent years in response to environmental issues. Thus, starting materials therefor are also required to be derived from biomass.

Accordingly, an object of the present invention is to provide an epoxy resin curing agent and the epoxy resin composition which can produce a cured product excellent in toughness, impact strength, adhesion, and chemical resistance. Another object of the present invention is to provide a novel amine compound which can be used in an epoxy resin curing agent and the like. A further object of the present invention is to provide an epoxy resin curing agent and a novel amine compound which can be produced using a starting material derived from biomass.

### Solution to Problem

The present inventors have found that the above objects can be attained by an epoxy resin curing agent containing an amine compound having a specific structure, or a modified product thereof.

Specifically, the present invention relates to the following aspects.
[1] An epoxy resin curing agent comprising at least one member selected from the group consisting of an amine compound (a) represented by the following general formula (1), and modified product (A) of the amine compound (a): 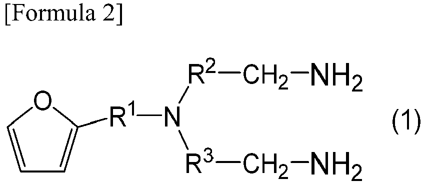 wherein, in the formula (1), R¹ is a linear alkylene group having 1 to 3 carbon atoms, and R² and R³ are each independently a linear or branched alkylene group having 2 to 4 carbon atoms.
[2] The epoxy resin curing agent according to [1], wherein R¹ is a methylene group, and R² and R³ are each independently an ethylene group or a 2-methylethylene group.
[3] The epoxy resin curing agent according to [1] or [2], wherein a biomass content is 30 mass% or more.
[4] The epoxy resin curing agent according to any one of [1] to [3], wherein the modified product (A) of the amine compound (a) comprises at least one member selected from the group consisting of the following (I) to (IV):
   (I) a Mannich reaction product of the amine compound (a), a phenolic compound, and an aldehyde compound,
   (II) a reaction product of the amine compound (a) and an unsaturated hydrocarbon compound,
   (III) a reaction product of the amine compound (a) and an epoxy compound having at least one epoxy group, and
   (IV) a reaction product of the amine compound (a) and a carboxylic acid or a derivative thereof.
[5] The epoxy resin curing agent according to any one of [1] to [4], wherein the modified product (A) of the amine compound (a) is a reaction product of the amine compound (a) and an epoxy compound having at least one epoxy group.
[6] The epoxy resin curing agent according to any one of [1] to [5], wherein the epoxy resin curing agent comprises no diluent or comprises a diluent in a content of less than 50 mass%.
[7] An epoxy resin composition comprising epoxy resin and the epoxy resin curing agent according to any one of [1] to [6].
[8] The epoxy resin composition according to [7], further comprising at least one additive selected from the group consisting of a coupling agent, a flame retardant, an antioxidant, a light stabilizer, a plasticizer, a diluent, a curing accelerator, a pigment, an inorganic filler, and an organic filler.
[9] The epoxy resin composition according to [7] or [8], wherein the epoxy resin comprises at least one member selected from the group consisting of epoxy resin having glycidyl groups derived from bisphenol A with a biomass content of 20% or more, and epoxy resin having glycidyl groups derived from 1,6-hexanediol with a biomass content of 100%.
[10] The epoxy resin composition according to any one of [7] to [9], wherein a biomass content is 40 mass% or more.
[11] The epoxy resin composition according to any one of [7] to [10], wherein the epoxy resin composition comprises no diluent or comprises a diluent in a content of less than 50 mass%.
[12] A paint comprising the epoxy resin composition according to any one of [7] to [11].
[13] A cured product obtained by curing the epoxy resin composition according to any one of [7] to [11].
[14] The cured product according to [13], wherein a biomass content is 40 mass% or more.
[15] The cured product according to [13] or [14], wherein a compression strength is 70 MPa or more.
[16] The cured product according to any one of [13] to [15], wherein a compression modulus of elasticity is 1700 MPa or more.
[17] The cured product according to any one of [13] to [16], wherein the cured product is a molded article.
[18] A method for producing an epoxy resin curing agent, comprising the step of hydrogenating a nitrile compound represented by the following general formula (2) to give amine compound (a) represented by the following general formula (1): 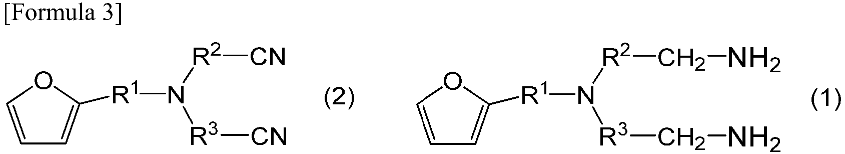 wherein, in the formula (1), R¹ is a linear alkylene group having 1 to 3 carbon atoms, and R² and R³ are each independently a linear or branched alkylene group having 2 to 4 carbon atoms, and in the formula (2), R¹ is a linear alkylene group having 1 to 3 carbon atoms, and R² and R³ are each independently a linear or branched alkylene group having 2 to 4 carbon atoms.
[19] An amine compound represented by the following general formula (1a): 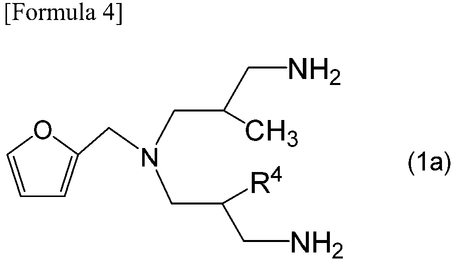 wherein, in the formula (1a), R⁴ is a hydrogen atom or a methyl group.

### Advantageous Effects of Invention

The present invention can provide an epoxy resin curing agent and an epoxy resin composition which can produce a cured product excellent in toughness, impact strength, adhesion, and chemical resistance. The present invention can also provide a novel amine compound which can be used in an epoxy resin curing agent and the like. The present invention can further provide an epoxy resin curing agent and a novel amine compound which can be produced using a starting material derived from biomass.

### Description of Embodiments

### [Epoxy resin curing agent]

The epoxy resin curing agent of the present invention comprises at least one member selected from the group consisting of an amine compound (a) represented by the general formula (1) given below, and a modified product (A) of the amine compound (a).

The "modified product of the amine compound (a)" used herein is a product obtained by modifying the amine compound (a) with another component, and means a reaction product of the amine compound (a) and another component as well as a reaction composition containing a by-product, an unreacted starting material, and the like, in addition to the reaction product. The modified product (A) of the amine compound (a) will be mentioned later. 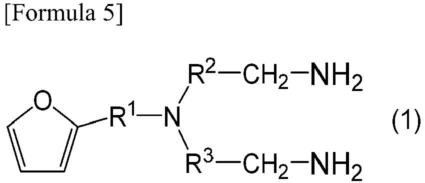 wherein, in the formula (1), R¹ is a linear alkylene group having 1 to 3 carbon atoms, and R² and R³ are each independently a linear or branched alkylene group having 2 to 4 carbon atoms.

The use of the above epoxy resin curing agent as a component of an epoxy resin composition can produce a cured product excellent in toughness, impact strength, adhesion, and chemical resistance. Hereinafter, the epoxy resin curing agent of the present invention is also simply referred to as the "curing agent of the present invention".

The reason why the use of the epoxy resin curing agent of the present invention produces the above effects is not certain and however, is presumably as described below.

The cured product of the present invention has a furan ring (aromatic ring) in a side chain and is considered to have thereby improved toughness, impact strength, and chemical resistance. Furthermore, the cured product of the present invention tends to have a relatively low viscosity and is considered to have thereby improved adhesion owing to an anchor effect.

For these reasons, the use of the epoxy resin curing agent of the present invention as a component of an epoxy resin composition is considered to be able to produce a cured product excellent in toughness, impact strength, adhesion, and chemical resistance, and to also attain an excellent curing rate.

The biomass content of the epoxy resin curing agent of the present invention is preferably 30 mass% or more, more preferably 50 mass% or more, further preferably 70 mass% or more, still further preferably 90 mass% or more. The upper limit is not limited and is 100 mass% or less, still further preferably 100 mass%. The amine compound (a) contained in the epoxy resin curing agent can be obtained using biomass-derived furfural and biomass-derived acrylonitrile as starting materials, as mentioned later, and the biomass content can be adjusted to the above range by using a biomass-derived starting material including these or other than furfural or acrylonitrile. It should be noted that the biomass content is the proportion of carbon of a biomass-derived component among all carbon atoms in the curing agent.

### <Amine compound (a) represented by general formula (1)>

Hereinafter, the amine compound (a) represented by the general formula (1) is also simply referred to as the "amine compound (a)".

In the general formula (1), R¹ is a linear alkylene group having 1 to 3 carbon atoms, preferably a linear alkylene group having 1 or 2 carbon atoms, more preferably an alkylene group having 1 carbon atom (methylene group).

In the general formula (1), R² and R³ are each independently a linear or branched alkylene group having 2 to 4 carbon atoms, preferably a linear or branched alkylene group having 2 or 3 carbon atoms, more preferably a linear alkylene group having 2 carbon atoms or a branched alkylene group having 3 carbon atoms, further preferably an ethylene group or a 2-methylethylene group. R² and R³ may be the same or may be different.

Thus, the amine compound (a) represented by the general formula (1) is preferably an amine compound represented by the formula (1b) given below.

The amine compound (a) represented by the general formula (1) is an amine compound represented by the formula (1b), whereby the epoxy resin curing agent comprising the amine compound (a) can produce a cured product excellent in toughness, impact strength, adhesion, and chemical resistance. 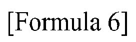 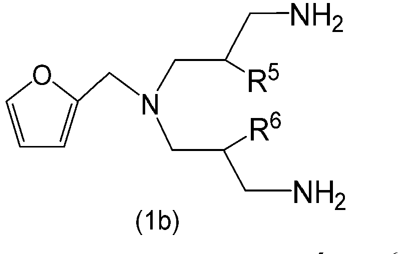 wherein, in the formula (1b), R⁵ and R⁶ are each independently a hydrogen atom or a methyl group.

In the general formula (1b), R⁵ and R⁶ are each independently a hydrogen atom or a methyl group, preferably a hydrogen atom.

In the general formula (1b), when R⁵ is a hydrogen atom, R⁶ is a hydrogen atom or a methyl group, preferably a hydrogen atom.

In the general formula (1b), when R⁵ is a methyl group, R⁶ is a hydrogen atom or a methyl group, preferably a hydrogen atom.

Thus, the amine compound (a) represented by the general formula (1) is more preferably an amine compound represented by the formula (1c) given below or an amine compound represented by the general formula (1a) given below, further preferably an amine compound represented by the formula (1c) given below.

The amine compound (a) represented by the general formula (1) is an amine compound represented by the formula (1c) or an amine compound represented by the formula (1a), whereby the epoxy resin curing agent comprising the amine compound (a) can produce a cured product excellent in toughness, impact strength, adhesion, and chemical resistance. 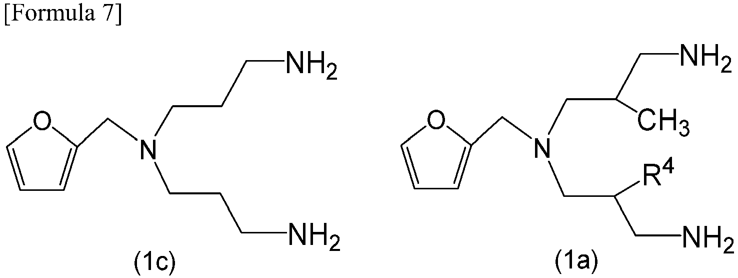 wherein, in the formula (1a), R⁴ is a hydrogen atom or a methyl group.

The active hydrogen equivalent weight of the amine compound (a) is preferably 30 or more, more preferably 40 or more, further preferably 50 or more, and preferably 80 or less, more preferably 70 or less, further preferably 65 or less, from the viewpoint of improvement in chemical resistance of the resulting cured product. The active hydrogen equivalent weight (hereinafter, also referred to as "AHEW") of the amine compound (a) is the mass per mol of active hydrogen of the amine compound (a).

AHEW of the amine compound (a) can be obtained, for example, by measuring an amine value by a dropwise addition method and calculating it from the amine value. Alternatively, AHEW may be calculated from a theoretical value when the amine compound (a) consists of a single compound and has a known structure.

### (Method for producing amine compound (a))

A method for producing the amine compound (a) is not particularly limited, and a known method may be used.

The amine compound (a) is preferably obtained by the following production method.

Specifically, the method for producing the amine compound (a) is preferably a production method comprising the step of hydrogenating a nitrile compound represented by the general formula (2) given below to give an amine compound (a) represented by the general formula (1) given below.

It should be noted that the present production method constitutes one step of a suitable form in a method for producing the epoxy resin curing agent mentioned later. Specifically, a suitable method for producing the epoxy resin curing agent according to the present invention is a method for producing an epoxy resin curing agent, comprising the step of hydrogenating a nitrile compound represented by the general formula (2) given below to give an amine compound (a) represented by the general formula (1) given below.

Furthermore, the present production method is a suitable method for producing an amine compound represented by the general formula (1a) mentioned later. 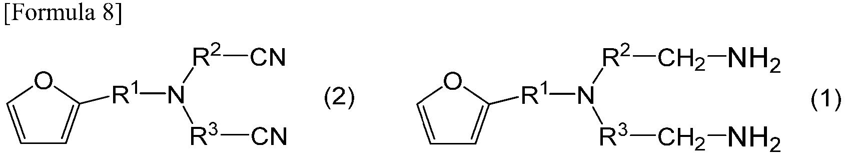 wherein, in the formula (1), R¹ is a linear alkylene group having 1 to 3 carbon atoms, and R² and R³ are each independently a linear or branched alkylene group having 2 to 4 carbon atoms, and in the formula (2), R¹ is a linear alkylene group having 1 to 3 carbon atoms, and R² and R³ are each independently a linear or branched alkylene group having 2 to 4 carbon atoms.

### (Nitrile compound hydrogenation step)

This step is the step of hydrogenating a nitrile compound represented by the formula (2) to give an amine compound (a) represented by the formula (1).

The reaction of this step is represented by the following formula. 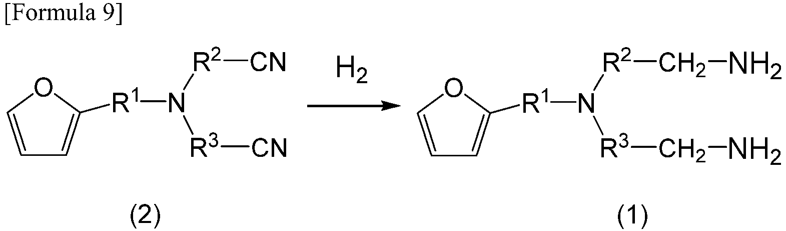 wherein, in the formula (1), R¹ is a linear alkylene group having 1 to 3 carbon atoms, and R² and R³ are each independently a linear or branched alkylene group having 2 to 4 carbon atoms, and in the formula (2), R¹ is a linear alkylene group having 1 to 3 carbon atoms, and R² and R³ are each independently a linear or branched alkylene group having 2 to 4 carbon atoms.

Hereinafter, the reaction of this step will be more specifically described.

The form of the hydrogenation reaction in this step is not particularly limited and may be any of a batch, semicontinuous, and continuous methods, for example.

The reaction temperature of the hydrogenation reaction is preferably 80 to 180°C, more preferably 100 to 140°C from the viewpoint of a reaction rate, further preferably 110 to 130°C from the viewpoint of selectivity and a reaction rate.

The pressure of hydrogen in the hydrogenation reaction is preferably 1 to 20 MPa, more preferably 5 to 10 MPa.

The hydrogenation reaction can be performed without a solvent and may be performed using a solvent.

Examples of the solvent in the hydrogenation reaction include water; aromatic compounds such as benzene, o-dichlorobenzene, toluene, and xylene; hydrocarbons such as hexane, heptane, and cyclohexane; alcohols such as methanol, ethanol, isopropyl alcohol, t-butyl alcohol, ethylene glycol, and diethylene glycol; ethers such as dioxane, tetrahydrofuran, dimethoxyethane, and diglyme; and mixtures thereof.

The amount of the solvent used in the hydrogenation reaction is preferably 0 to 30 times, more preferably 0 to 20 times the mass of the nitrile compound.

A catalyst is used in the hydrogenation reaction. The catalyst for use in the hydrogenation reaction is not particularly limited as long as the catalyst is usually used in the hydrogenation of a nitrile compound. The catalyst preferably contains at least one member selected from the group consisting of metals of groups 8 to 11 of the periodic table.

Specific examples thereof include hydrogenation catalysts containing at least one member selected from the group consisting of iron, cobalt, nickel, copper, ruthenium, rhodium, palladium, silver, osmium, iridium, platinum, and gold.

The hydrogenation catalyst may be a solid catalyst or a homogeneous catalyst and is preferably a solid catalyst from the viewpoint of separability from a reaction product. Examples of the solid catalyst include non-supported metal catalysts and supported metal catalysts.

The non-supported metal catalyst is preferably a sponge metal catalyst such as sponge nickel, sponge cobalt, or sponge copper, or an oxide or colloid catalyst of platinum, palladium, rhodium, ruthenium, or the like.

Examples of the supported metal catalyst include catalysts of at least one of iron, cobalt, nickel, copper, ruthenium, rhodium, palladium, silver, osmium, iridium, platinum, and gold supported on a support such as magnesia, zirconia, seria, diatomaceous earth, activated carbon, alumina, silica, zeolite, or titania. The supported metal catalyst is preferably, for example, a supported copper catalyst such as a copper-chromium catalyst (Adkins catalyst), a copper-zinc catalyst, or a copper-iron, a supported platinum catalyst such as Pt/C or Pt/alumina, a supported palladium catalyst such as Pd/C or Pd/alumina, a supported ruthenium catalyst such as Ru/C or Ru/alumina, or a supported rhodium catalyst such as Rh/C or Rh/alumina.

Among these, a catalyst containing at least one member selected from the group consisting of nickel, cobalt, and ruthenium is more preferred from the viewpoint of reaction activity.

The amount of the hydrogenation catalyst used can be appropriately changed depending on the type of the catalyst and is preferably 0.1 to 100 parts by mass, more preferably 1 to 20 parts by mass, per 100 parts by mass of the starting material nitrile compound.

The amine compound (a) thus obtained is preferably purified by distillation. The distillation conditions can be appropriately adjusted depending on a pressure and a temperature in the distillation.

### (Unsaturated nitrile addition step)

The nitrile compound represented by the formula (2) for use in the nitrile compound hydrogenation step can be preferably synthesized by the following step.

This step is the step of adding an unsaturated nitrile compound represented by the formula (4) to an amine compound represented by the formula (3) to give a nitrile compound represented by the formula (2).

The reaction of this step is represented by the following formula. 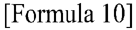 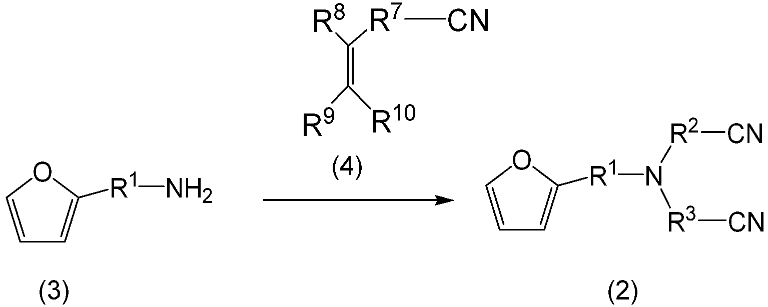 wherein R⁷ is a single bond or an alkylene group having 1 or 2 carbon atoms, R⁸, R⁹, and R¹⁰ are each independently a hydrogen atom or an alkyl group having 1 or 2 carbon atoms, the total number of carbon atoms in R⁷, R⁸, R⁹, and R¹⁰ is 0 to 2, R¹ is a linear alkylene group having 1 to 3 carbon atoms, and R² and R³ are each independently a linear or branched alkylene group having 2 to 4 carbon atoms.

The form of the unsaturated nitrile addition reaction is not particularly limited and may be any of a batch, semicontinuous, and continuous methods, for example.

The reaction temperature of the unsaturated nitrile addition reaction in this step is preferably 0°C to 150°C, more preferably 60°C to 120°C. The reaction pressure of the unsaturated nitrile addition reaction is not limited as long as the reaction suitably progresses. The reaction pressure is preferably 0 to 5 MPa, more preferably 0 to 1 MPa.

The unsaturated nitrile addition reaction may be performed without a catalyst or may be performed using a catalyst. The catalyst is preferably a base catalyst.

The base catalyst for use in the unsaturated nitrile addition reaction is preferably an inorganic base.

The inorganic base is preferably at least one member selected from the group consisting of LiOH, NaOH, KOH, LiHCO₃, NaHO₃, KHO₃, Li₂CO₃, Na₂CO₃, K₂CO₃, Cs₂CO₃, Mg(OH)₂, Ca(OH)₂, Li₃PO₄, Na₃PO₄, K₃PO₄, t-BuONa, t-BuOK, and NaH, more preferably at least one member selected from the group consisting of NaOH and KOH.

The amount of the base catalyst is preferably 0.01 to 1 times, more preferably 0.01 to 0.1 times the mol of the starting material amine in this step. Although the base catalyst may be used in an amount exceeding 1 time the mol of the substrate, this is not preferred because the amount of the base as a waste is increased.

The unsaturated nitrile addition reaction may be performed without a solvent and may be performed using a solvent. Examples of the solvent include water; aromatic compounds such as benzene, o-dichlorobenzene, toluene, and xylene; hydrocarbons such as hexane, heptane, and cyclohexane; alcohols such as methanol, ethanol, isopropyl alcohol, t-butyl alcohol, ethylene glycol, and diethylene glycol; ethers such as dioxane, tetrahydrofuran, dimethoxyethane, and diglyme; and mixtures thereof.

The amount of the solvent used in the unsaturated nitrile addition reaction is preferably 0 to 30 times, more preferably 0 to 10 times the mass of the starting material amine in this step.

The amount of the unsaturated nitrile used in the unsaturated nitrile addition reaction is preferably 2 to 20, more preferably 2 to 4, in terms of a molar ratio to the starting material amine in this step [unsaturated nitrile/amine].

### (Production method using furfural as starting material (reductive amination step))

The method for producing the amine compound (a) is further preferably the method for producing the amine compound (a), wherein the amine compound represented by the formula (3) is an amine compound obtained from furfural as a starting material. The amine compound obtained here can be subjected as an intermediate material to the above unsaturated nitrile addition step and the above hydrogenation step to give the amine compound (a) of interest. The use of furfural as a starting material is preferred because the biomass content of the resulting amine compound (a) can be elevated.

The method for obtaining the amine compound represented by the formula (3) from furfural as a starting material is not limited and can be a method by which the amine compound represented by the formula (3) is finally obtained. Among others, a preferred method will be described below in detail.

In the case of using the biomass-derived starting material furfural as a starting material in the present production method, the present production method comprises the step of reductively aminizing furfural to give furfurylamine (amine compound represented by the formula (3a) given below) which is the amine compound represented by the formula (3).

The reaction of this step is represented by the following formula.

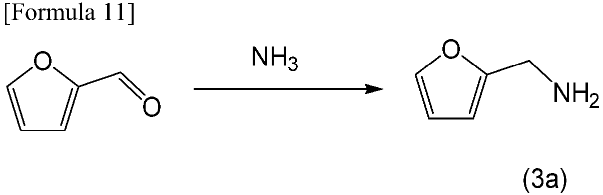

An aldehyde group of furfural is reductively aminized to give furfurylamine.

The reductive amination reaction is preferably carried out using ammonia water as an amine source and Rh/Al₂O₃ as a catalyst under conditions involving a reaction temperature of 80°C and a hydrogen pressure of 2 MPa.

### (Production method using biomass-derived acrylonitrile as starting material (acrylonitrile addition step))

The method for producing the amine compound (a) is further preferably the method for producing the amine compound (a), wherein the nitrile compound represented by the formula (2) is a nitrile compound obtained from biomass-derived acrylonitrile as a starting material. The nitrile compound obtained here can be subjected as an intermediate material to the above hydrogenation step to give the amine compound (a) of interest. The use of biomass-derived acrylonitrile as a starting material is preferred because the biomass content of the resulting amine compound (a) can be elevated.

The method for obtaining the nitrile compound represented by the formula (2) from biomass-derived acrylonitrile as a starting material is not limited and can be a method by which the nitrile compound represented by the formula (2) is finally obtained. Among others, a preferred method will be described below in detail.

The present production method preferably comprises the step of adding acrylonitrile to an amine compound represented by the formula (3) to give a nitrile compound represented by the formula (2a). It should be noted that the nitrile compound represented by the formula (2a) is a compound in which each of R² and R³ in the nitrile compound represented by the formula (2) is an ethylene group.

The reaction of this step is represented by the following formula. 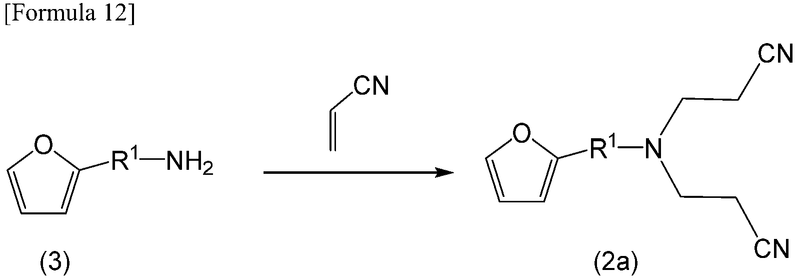 wherein R¹ is a linear alkylene group having 1 to 3 carbon atoms.

The form of the acrylonitrile addition reaction is not particularly limited and may be any of a batch, semicontinuous, and continuous methods, for example.

The reaction temperature of the acrylonitrile addition reaction in this step is preferably 0°C to 150°C, more preferably 60°C to 120°C. The reaction pressure of the acrylonitrile addition reaction is not limited as long as the reaction suitably progresses. The reaction pressure is preferably 0 to 5 MPa, more preferably 0 to 1 MPa.

The acrylonitrile addition reaction may be performed without a catalyst or may be performed using a catalyst. The catalyst is preferably a base catalyst.

The base catalyst for use in the acrylonitrile addition reaction is preferably an inorganic base.

The inorganic base is preferably at least one member selected from the group consisting of LiOH, NaOH, KOH, LiHCO₃, NaHO₃, KHO₃, Li₂CO₃, Na₂CO₃, K₂CO₃, Cs₂CO₃, Mg(OH)₂, Ca(OH)₂, Li₃PO₄, Na₃PO₄, K₃PO₄, t-BuONa, t-BuOK, and NaH, more preferably at least one member selected from the group consisting of NaOH and KOH.

The amount of the base catalyst is preferably 0.01 to 1 times, more preferably 0.01 to 0.1 times the mol of the starting material amine in this step. Although the base catalyst may be used in an amount exceeding 1 time the mol of the substrate, this is not preferred because the amount of the base as a waste is increased.

The acrylonitrile addition reaction may be performed without a solvent and may be performed using a solvent. Examples of the solvent include water; aromatic compounds such as benzene, o-dichlorobenzene, toluene, and xylene; hydrocarbons such as hexane, heptane, and cyclohexane; alcohols such as methanol, ethanol, isopropyl alcohol, t-butyl alcohol, ethylene glycol, and diethylene glycol; ethers such as dioxane, tetrahydrofuran, dimethoxyethane, and diglyme; and mixtures thereof.

The amount of the solvent used in the acrylonitrile addition reaction is preferably 0 to 30 times, more preferably 0 to 10 times the mass of the starting material amine in this step.

The amount of the acrylonitrile used in the acrylonitrile addition reaction is preferably 2 to 20, more preferably 2 to 4, in terms of a molar ratio to the starting material amine in this step [acrylonitrile/amine].

### <Modified product (A) of amine compound (a) represented by general formula (1)>

The epoxy resin curing agent of the present invention also includes an epoxy resin curing agent comprising a modified product (A) of the amine compound (a) represented by the general formula (1).

The modified product (A) is a modified product of the amine compound (a) represented by the general formula (1). The modified product (A) of the amine compound (a) preferably comprises at least one member selected from the group consisting of the following (I) to (IV) from the viewpoint of improvement in toughness, impact strength, adhesion, and chemical resistance of the resulting cured product:
(I) a Mannich reaction product of the amine compound (a), a phenolic compound, and an aldehyde compound,
(II) a reaction product of the amine compound (a) and an unsaturated hydrocarbon compound,
(III) a reaction product of the amine compound (a) and an epoxy compound having at least one epoxy group, and
(IV) a reaction product of the amine compound (a) and a carboxylic acid or a derivative thereof.

The "reaction product" used herein is a product obtained through the reaction of the amine compound (a) and another component, and means a reaction product (adduct) of the amine compound (a) and another component as well as a reaction composition containing a by-product, an unreacted starting material, and the like, in addition to the reaction product.

### ((I) Mannich reaction product of amine compound (a), phenolic compound, and aldehyde compound)

The modified product (I) is a Mannich reaction product of the amine compound (a), a phenolic compound, and an aldehyde compound.

Examples of the phenolic compound for use in the modified product (I) include phenol, alkylphenol, alkenylphenol, terpenephenol, and naturally produced phenolic compounds such as cardanol. The number of carbon atoms in the alkyl group of the alkylphenol is preferably 1 to 24, more preferably 1 to 18, and the number of carbon atoms in the alkenyl group of the alkenylphenol is preferably 2 to 24, more preferably 2 to 18, from the viewpoint of improvement in toughness, impact strength, adhesion, and chemical resistance of the resulting cured product.

Specific examples of the phenolic compound include phenol, cresol, ethylphenol (p-ethylphenol, etc.), isopropylphenol (o-isopropylphenol, p-isopropylphenol, etc.), butylphenol (p-tert-butylphenol, p-sec-butylphenol, o-tert-butylphenol, o-sec-butylphenol, etc.), amylphenol (p-tert-amylphenol, o-tert-amylphenol, etc.), p-octylphenol, nonylphenol, p-cumylphenol, decylphenol, undecylphenol, p-dodecylphenol, tridecylphenol, tetradecylphenol, pentadecylphenol, pentadecenylphenol, pentadecadienylphenol, pentadecatrienylphenol, hexadecylphenol, heptadecylphenol, octadecylphenol, octadecenylphenol, terpenephenol, and cardanol. One of these compounds can be used singly, or two or more thereof can be used in combination.

Among those described above, the phenolic compound for use in the modified product (I) is preferably at least one member selected from the group consisting of phenol, cresol, p-tert-butylphenol, nonylphenol, and cardanol, more preferably phenol, from the viewpoint of reactivity with the amine compound (a) and from the viewpoint of improvement in toughness, impact strength, adhesion, and chemical resistance of the resulting cured product.

Examples of the aldehyde compound for use in the modified product (I) include formaldehyde; formaldehyde-releasing compounds such as trioxane and paraformaldehyde; and other aldehydes such as benzaldehyde. Among them, the aldehyde compound is preferably at least one member selected from the group consisting of formaldehyde and a formaldehyde-releasing compound. Among them, an aqueous formaldehyde solution is more preferably used from the viewpoint of workability in the Mannich reaction.

A method for producing the modified product (I) is not particularly limited, and a known method can be used. Examples thereof include a method of adding the aldehyde compound or a solution thereof to a mixture of the amine compound (a) and the phenolic compound by dropwise addition or the like at preferably 80°C or lower, more preferably 60°C or lower, raising the temperature to preferably 80 to 180°C, more preferably 90 to 150°C, after the completion of the addition, and reacting the mixture for preferably 0.5 to 12 hours while removing a distillate from the reaction system.

The reaction molar ratio among the amine compound (a), the phenolic compound, and the aldehyde compound in the Mannich reaction is not particularly limited as long as the ratio produces a modified product (I) containing an amino group having active hydrogen. The following ranges are preferred from the viewpoint of reactivity with the amine compound (a) and from the viewpoint of improvement in toughness, impact strength, adhesion, and chemical resistance of the resulting cured product.

The aldehyde compound is used in the range of preferably 0.3 to 2 mol, more preferably 0.5 to 1.5 mol, per mol of the amine compound (a). Furthermore, the phenolic compound is used in the range of preferably 0.3 to 2 mol, more preferably 0.5 to 1.5 mol, per mol of the amine compound (a).

### ((II) Reaction product of amine compound (a) and unsaturated hydrocarbon compound)

The modified product (II) is a reaction product of the amine compound (a) and an unsaturated hydrocarbon compound.

The unsaturated hydrocarbon compound for use in the modified product (II) is preferably an unsaturated hydrocarbon compound having 2 to 10 carbon atoms from the viewpoint of reactivity with the amine compound (a) and from the viewpoint of improvement in toughness, impact strength, adhesion, and chemical resistance of the resulting cured product.

Examples of the unsaturated hydrocarbon compound include unsaturated aliphatic hydrocarbon compounds having 2 to 10 carbon atoms, and aromatic hydrocarbon compounds having an ethylenic unsaturated bond having 2 to 10 carbon atoms, and include at least one member selected from the group consisting of ethylene, propylene, butene, pentene, hexene, heptene, octene, nonene, decene, isobutylene, 2-pentene, 3-methyl-1-butene, 2-methyl-2-butene, 2,3-dimethyl-2-butene, cyclohexene, cyclohexadiene, styrene, and divinylbenzene.

Among those described above, the unsaturated hydrocarbon compound is preferably an aromatic hydrocarbon compound having an ethylenic unsaturated bond, such as styrene or divinylbenzene, more preferably styrene, from the viewpoint of reactivity with the amine compound (a) and from the viewpoint of improvement in toughness, impact strength, adhesion, and chemical resistance of the resulting cured product.

A method for producing the modified product (II) is not particularly limited, and a known method can be used. The modified product is obtained, for example, through the addition reaction of the amine compound (a) and the unsaturated hydrocarbon compound at usually 25 to 150°C, preferably 60 to 150°C, in the presence of a basic catalyst such as lithium amide. More specifically, examples thereof include a method of adding the unsaturated hydrocarbon compound or a solution thereof to a mixture of the amine compound (a) and a basic catalyst by dropwise addition or the like at preferably 80°C or lower, more preferably 60°C or lower, raising the temperature to preferably 60 to 150°C, more preferably 90 to 150°C, after the completion of the addition, and reacting the mixture for preferably 0.5 to 12 hours.

The reaction molar ratio between the amine compound (a) and the unsaturated hydrocarbon compound in the addition reaction is not particularly limited as long as the ratio produces a modified product (II) containing an amino group having active hydrogen. The ratio is in the range of preferably 0.1 to 5.0 mol, more preferably 0.4 to 3.0 mol, further preferably 0.5 to 1.5 mol, still further preferably 0.8 to 1.2 mol, per mol of the amine compound (a).

### ((III) Reaction product of amine compound (a) and epoxy compound having at least one epoxy group)

The modified product (III) is a reaction product of the amine compound (a) and an epoxy compound having at least one epoxy group.

The epoxy compound for use in the modified product (III) can be a compound having at least one epoxy group and is more preferably a compound having two or more epoxy groups.

Examples of the epoxy compound for use in the modified product (III) include epichlorohydrin, butyl glycidyl ether, neopentyl glycol diglycidyl ether, 1,3-propanediol diglycidyl ether, 1,4-butanediol diglycidyl ether, 1,6-hexanediol diglycidyl ether, biphenol diglycidyl ether, dihydroxynaphthalene diglycidyl ether, dihydroxyanthracene diglycidyl ether, triglycidyl isocyanurate, tetraglycidyl glycol uril, polyfunctional epoxy resin having glycidyl groups derived from metaxylylenediamine, polyfunctional epoxy resin having glycidyl groups derived from 1,3-bis(aminomethyl)cyclohexane, polyfunctional epoxy resin having glycidyl groups derived from diaminodiphenylmethane, polyfunctional epoxy resin having glycidyl groups derived from paraaminophenol, polyfunctional epoxy resin having glycidyl groups derived from bisphenol A, polyfunctional epoxy resin having glycidyl groups derived from bisphenol F, polyfunctional epoxy resin having glycidyl groups derived from phenol novolac, and polyfunctional epoxy resin having two or more glycidyl groups derived from resorcinol, one or two or more of which can be used.

Among those described above, the epoxy compound is preferably an epoxy compound containing an aromatic ring or an alicyclic structure in a molecule, more preferably an epoxy compound containing an aromatic ring in a molecule, further preferably polyfunctional epoxy resin having glycidyl groups derived from bisphenol A, from the viewpoint of economic performance and from the viewpoint of improvement in toughness, impact strength, adhesion, and chemical resistance of the resulting cured product.

A method for producing the modified product (III) is not particularly limited, and a known method can be used. The modified product is obtained, for example, through the addition reaction of the amine compound (a) and the epoxy compound under a heating condition. More specifically, examples thereof include a method of adding the epoxy compound or a solution thereof to the amine compound (a) or a solution thereof with stirring by dropwise addition or the like at preferably 80°C or lower, more preferably 60°C or lower, raising the temperature to preferably 60 to 150°C, more preferably 70 to 120°C, after the completion of the addition, and reacting the mixture for preferably 0.5 to 12 hours.

The reaction molar ratio between the amine compound (a) and the epoxy compound in the addition reaction is not particularly limited as long as the ratio produces a modified product (III) containing an amino group having active hydrogen. An excessive amount of the amine compound (a) is preferably used based on the epoxy equivalent weight of the epoxy compound. Specifically, the amine compound (a) and the epoxy compound are reacted at a ratio such that the number of active hydrogen atoms in the amine compound (a)/the number of epoxy groups in the epoxy compound is preferably 4/1 to 50/1, more preferably 8/1 to 20/1.

### ((IV) Reaction product of amine compound (a) and carboxylic acid or derivative thereof)

The modified product (IV) is a reaction product of the amine compound (a) and a carboxylic acid or a derivative thereof.

Examples of the carboxylic acid or the derivative thereof for use in the modified product (IV) include unsaturated carboxylic acids such as acrylic acid, methacrylic acid, α-ethylacrylic acid, α-propylacrylic acid, α-isopropylacrylic acid, α-n-butylacrylic acid, α-t-butylacrylic acid, α-pentylacrylic acid, α-phenylacrylic acid, α-benzylacrylic acid, crotonic acid, 2-pentenoic acid, 2-hexenoic acid, 4-methyl-2-pentenoic acid, 2-heptenoic acid, 4-methyl-2-hexenoic acid, 5-methyl-2-hexenoic acid, 4,4-dimethyl-2-pentenoic acid, 4-phenyl-2-butenoic acid, cinnamic acid, o-methylcinnamic acid, m-methylcinnamic acid, p-methylcinnamic acid, and 2-octenoic acid, and their esters, amides, acid anhydrides, and acid chlorides.

Among those described above, the carboxylic acid or the derivative thereof is preferably at least one member selected from the group consisting of acrylic acid, methacrylic acid, crotonic acid, and their derivatives, more preferably at least one member selected from the group consisting of acrylic acid, methacrylic acid, crotonic acid, and their alkyl esters, further preferably at least one member selected from the group consisting of acrylic acid, methacrylic acid, and their alkyl esters, still further preferably acrylic acid alkyl ester, still further preferably methyl acrylate.

A method for producing the modified product (IV) is not particularly limited, and a known method can be used. The modified product is obtained, for example, through the reaction of the amine compound (a) and the carboxylic acid or the derivative thereof under a heating condition. More specifically, examples thereof include a method of adding the carboxylic acid or the derivative thereof to the amine compound (a) or a solution thereof with stirring by dropwise addition or the like at preferably 80°C or lower, more preferably 60°C or lower, raising the temperature to preferably 60 to 150°C after the completion of the addition, and reacting the mixture for preferably 0.5 to 12 hours.

The reaction molar ratio between the amine compound (a) and the carboxylic acid or the derivative thereof in the addition reaction is not particularly limited as long as the ratio produces a modified product (IV) containing an amino group having active hydrogen. The ratio is in the range of preferably 0.3 to 1.0 mol, more preferably 0.6 to 1.0 mol, per mol of the amine compound (a).

### (Production, etc. of modified product (A))

The modified products (I) to (IV) may be performed in a solvent or may be performed without a solvent. Furthermore, the reaction with the amine compound (a) is preferably performed in an inert atmosphere of nitrogen gas or the like.

One of or two or more in combination of the modified products of the amine compound (a) can be used as the modified product (A).

Among those described above, the modified product (A) preferably comprises (III) a reaction product of the amine compound (a) and an epoxy compound having at least one epoxy group, from the viewpoint of improvement in toughness, impact strength, adhesion, and chemical resistance of the resulting cured product. Furthermore, the epoxy compound is preferably a compound having two or more epoxy groups, more preferably an epoxy compound containing an aromatic ring or an alicyclic structure in a molecule and having two or more epoxy groups, further preferably an epoxy compound containing an aromatic ring in a molecule and having two or more epoxy groups, still further preferably polyfunctional epoxy resin having glycidyl groups derived from bisphenol A.

The content of the reaction product (III) of the amine compound (a) and the epoxy compound having at least one epoxy group in the modified product (A) is preferably 50 mass% or more, more preferably 60 mass% or more, further preferably 70 mass% or more, still further preferably 80 mass% or more, still further preferably 90 mass% or more, still further preferably 95 mass% or more, and 100 mass% or less, from the viewpoint of improvement in toughness, impact strength, adhesion, and chemical resistance of the resulting cured product.

The active hydrogen equivalent weight of the modified product (A) is preferably 60 or more, more preferably 70 or more, further preferably 75 or more, and preferably 170 or less, more preferably 150 or less, further preferably 120 or less, from the viewpoint of improvement in chemical resistance of the resulting cured product.

AHEW of the modified product (A) can be obtained, for example, by measuring an amine value by a dropwise addition method and calculating it from the amine value. Alternatively, AHEW may be calculated from a theoretical value when the modified product (A) consists of a single compound and has a known structure.

### <Diluent>

The epoxy resin curing agent of the present invention may be further supplemented with a diluent and may be supplemented with a known non-reactive diluent or the like as the diluent, without impairing the advantageous effects of the present invention.

Examples of the non-reactive diluent include benzyl alcohol, furfuryl alcohol, tetrafurfuryl alcohol, and aromatic hydrocarbon formaldehyde resin, one or two or more of which can be used.

The aromatic hydrocarbon formaldehyde resin is a resin obtained through the reaction of aromatic hydrocarbon and formaldehyde. Examples thereof include toluene formaldehyde resin obtained through the reaction of toluene and formaldehyde, xylene formaldehyde resin obtained through the reaction of xylene and formaldehyde, mesitylene formaldehyde resin obtained through the reaction of mesitylene and formaldehyde, and pseudocumene formaldehyde resin obtained through the reaction of pseudocumene and formaldehyde.

Examples of a commercially available product of the aromatic hydrocarbon formaldehyde resin include "NIKANOL Y-50", "NIKANOL Y-100", "NIKANOL Y-300", "NIKANOL Y-1000", "NIKANOL L", "NIKANOL LL", "NIKANOL LLL", "NIKANOL G", "NIKANOL H", and "NIKANOL H-80", which are xylene formaldehyde resin manufactured by Fudow Co., Ltd.

Among those described above, the non-reactive diluent is preferably at least one member selected from the group consisting of benzyl alcohol and aromatic hydrocarbon formaldehyde resin, more preferably is at least one member selected from the group consisting of benzyl alcohol and xylene formaldehyde resin, further preferably comprises benzyl alcohol, and still further preferably is benzyl alcohol.

The content of the diluent is preferably 1 to 99 mass%, more preferably 10 to 90 mass%, further preferably 20 to 70 mass%, still further preferably 30 to 60 mass%, still further preferably 35 to 50 mass%, based on the total amount of the epoxy resin curing agent.

The content of the non-reactive diluent is preferably 1 to 99 mass%, more preferably 10 to 90 mass%, further preferably 20 to 70 mass%, still further preferably 30 to 60 mass%, still further preferably 35 to 50 mass%, based on the total amount of the epoxy resin curing agent.

It should be noted that the amine compound (a) and the modified product (A) thereof have a low viscosity and therefore produce a cured product having favorable properties substantially without the use of the diluent, when used as an epoxy resin curing agent. Thus, the epoxy resin curing agent of the present invention preferably comprises no diluent or comprises a diluent in the content of less than 50 mass%. Such a decreased amount of the diluent used can elevate the concentration of the amine compound (a) produced using a biomass-derived starting material in the curing agent and by extension, can improve the biomass content of an epoxy resin composition.

On the other hand, polyaminoamide-based curing agents heretofore used have a high viscosity and therefore require a diluent and have the difficulty in improving the biomass content of an epoxy resin composition.

The total content of the amine compound (a) and the modified product (A) thereof used in the epoxy resin curing agent of the present invention is not particularly limited as long as the upper limit is 100 mass% or less. The total content is preferably 1 to 99 mass%, more preferably 10 to 90 mass%, further preferably 30 to 80 mass%, still further preferably 40 to 70 mass%, still further preferably 50 to 65 mass%, based on the total amount of the epoxy resin curing agent.

The epoxy resin curing agent of the present invention may further contain another known curing agent component without impairing the advantageous effects of the present invention. The curing agent component described herein means a component that is contained in the epoxy resin curing agent and has two or more active hydrogen atoms capable of reacting with epoxy groups in epoxy resin.

Examples of another curing agent component include amine-based curing agents other than the amine compound (a) and the modified product (A) thereof, phenol-based curing agents, and acid anhydride-based curing agents. An amine-based curing agent is preferred from the viewpoint of fast curing.

In the epoxy resin curing agent of the present invention, it is preferred to use only the amine compound (a) and the modified product (A) thereof as a curing agent component.

The epoxy resin curing agent of the present invention may be further supplemented with a known curing accelerator or the like without impairing the advantageous effects of the present invention.

Examples of the curing accelerator include phenolic compounds, organic acids, organic acid salts, tertiary amines, quaternary ammonium salts, imidazoles, organic phosphorus compounds, quaternary phosphonium salts, diazabicycloalkenes, organic metal salt compounds, boron compounds, and metal halides.

The active hydrogen equivalent weight of the epoxy resin curing agent is preferably 70 or more, more preferably 80 or more, further preferably 100 or more, still further preferably 140 or more, still further preferably 200 or more, and preferably 300 or less, more preferably 280 or less, further preferably 250 or less, from the viewpoint of improvement in chemical resistance of the resulting cured product. The active hydrogen equivalent weight (hereinafter, also referred to as "AHEW") of the epoxy resin curing agent is the mass of the whole epoxy resin curing agent including the non-reactive diluent and the like per mol of active hydrogen of the epoxy resin curing agent.

### [Method for producing epoxy resin curing agent]

A method for producing the epoxy resin curing agent of the present invention is not particularly limited and can be appropriately selected depending on a usage pattern, an apparatus used, the types and blending proportions of blended components, and so on. The epoxy resin curing agent can be produced, for example, by blending and mixing at least one member selected from the group consisting of the amine compound (a) and the modified product (A) of the amine compound (a), and the non-reactive diluent and the like which are optionally used. Alternatively, an epoxy resin composition may be produced by mixing the individual components contained in the epoxy resin curing agent, and epoxy resin at the same time during the production of the epoxy resin composition.

As described above, the epoxy resin curing agent of the present invention may be produced by any method as long as the configuration described above is satisfied. The epoxy resin curing agent is preferably produced by the following method, and the following production method is also included in the present invention.

The method for producing the epoxy resin curing agent of the present invention comprises the step of hydrogenating a nitrile compound represented by the following general formula (2) to give an amine compound (a) represented by the following general formula (1): 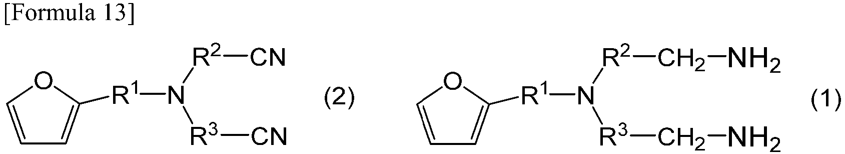 wherein, in the formula (1), R¹ is a linear alkylene group having 1 to 3 carbon atoms, and R² and R³ are each independently a linear or branched alkylene group having 2 to 4 carbon atoms, and in the formula (2), R¹ is a linear alkylene group having 1 to 3 carbon atoms, and R² and R³ are each independently a linear or branched alkylene group having 2 to 4 carbon atoms.

The "step to give an amine compound (a) represented by the formula (1)" in the present production method is the same as the "step of hydrogenating a nitrile compound represented by the general formula (2) to give an amine compound (a) represented by the general formula (1)" described in the above section (Method for producing amine compound (a)), and preferred conditions are also the same thereas. Furthermore, all of other steps necessary for obtaining the amine compound (a) are the same as those described in the above section (Method for producing amine compound (a)). Hereinafter, description will be made.

The nitrile compound represented by the formula (2) for use in the nitrile compound hydrogenation step can be preferably synthesized by the following step.

This step is the step of adding an unsaturated nitrile compound represented by the formula (4) to an amine compound represented by the formula (3) to give a nitrile compound represented by the formula (2).

The reaction of this step is represented by the following formula. 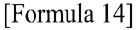 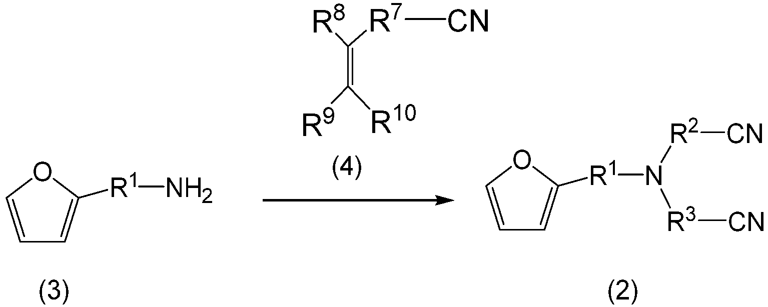 wherein R⁷ is a single bond or an alkylene group having 1 or 2 carbon atoms, R⁸, R⁹, and R¹⁰ are each independently a hydrogen atom or an alkyl group having 1 or 2 carbon atoms, the total number of carbon atoms in R⁷, R⁸, R⁹, and R¹⁰ is 0 to 2, R¹ is a linear alkylene group having 1 to 3 carbon atoms, and R² and R³ are each independently a linear or branched alkylene group having 2 to 4 carbon atoms.

In the case of using the biomass-derived starting material furfural as a starting material in the present production method, the present production method comprises the step of reductively aminizing furfural to give furfurylamine (amine compound represented by the formula (3a) given below) which is the amine compound represented by the formula (3).

The reaction of this step is represented by the following formula.

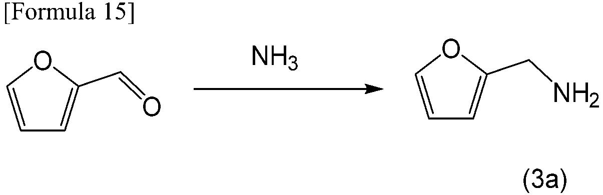

In the case of using the biomass-derived starting material acrylonitrile as a starting material in the present production method, the present production method preferably comprises the step of adding acrylonitrile to an amine compound represented by the formula (3) to give a nitrile compound represented by the formula (2a).

The reaction of this step is represented by the following formula. 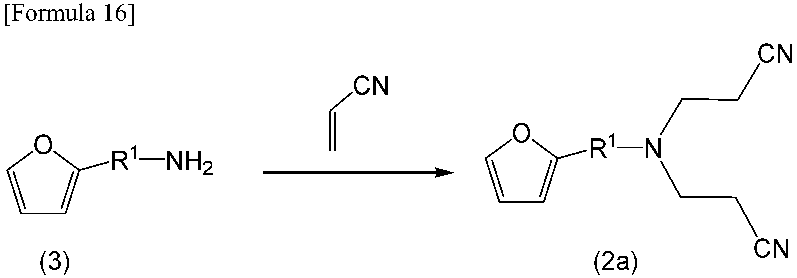 wherein R¹ is a linear alkylene group having 1 to 3 carbon atoms.

In the production method of the present invention, the amine compound (a) obtained as described above may be mixed with the non-reactive diluent and the like to prepare an epoxy resin curing agent; the amine compound (a) may be modified by the above method to give a modified product (A), and the modified product (A) can then be mixed with the non-reactive diluent and the like to prepare an epoxy resin curing agent; or the amine compound (a) and the modified product (A) may be mixed with the non-reactive diluent and the like to prepare an epoxy resin curing agent.

### [Epoxy resin composition]

The epoxy resin composition of the present invention comprises epoxy resin and the epoxy resin curing agent. The epoxy resin composition of the present invention comprises the epoxy resin curing agent and can therefore produce a cured product excellent in toughness, impact strength, adhesion, and chemical resistance.

The biomass content of the epoxy resin composition of the present invention is preferably 40 mass% or more. The upper limit is not limited and is 100 mass% or less, still further preferably 100 mass%. The biomass content can be adjusted to the above range by producing the epoxy resin curing agent contained in the epoxy resin composition using biomass-derived starting materials, as mentioned above, and further using epoxy resin having a high biomass content mentioned later. It should be noted that the biomass content is the proportion of carbon of a biomass-derived component among all carbon atoms in a cured product.

### <Epoxy resin>

The main agent epoxy resin in the epoxy resin composition may be any of a saturated or unsaturated aliphatic compound, an alicyclic compound, an aromatic compound, and a heterocyclic compound. The epoxy resin preferably contains an aromatic ring or an alicyclic structure in a molecule from the viewpoint of curability and improvement in appearance, water resistance, impact resistance, and adhesion with a base material of the resulting coating film.

Specific examples of the epoxy resin include at least one resin selected from the group consisting of epoxy resin having glycidyl groups derived from metaxylylenediamine, epoxy resin having glycidyl groups derived from paraxylylenediamine, epoxy resin having glycidyl groups derived from 1,3-bis(aminomethyl)cyclohexane, epoxy resin having glycidyl groups derived from 1,4-bis(aminomethyl)cyclohexane, epoxy resin having glycidyl groups derived from diaminodiphenylmethane, epoxy resin having glycidyl groups derived from paraaminophenol, epoxy resin having glycidyl groups derived from bisphenol A, epoxy resin having glycidyl groups derived from bisphenol F, epoxy resin having glycidyl groups derived from phenol novolac, and epoxy resin having glycidyl groups derived from resorcinol. Two or more of the above epoxy resins may be mixed for use.

Among those described above, the epoxy resin is preferably epoxy resin having at least one main component selected from the group consisting of epoxy resin having glycidyl groups derived from metaxylylenediamine, epoxy resin having glycidyl groups derived from paraxylylenediamine, epoxy resin having glycidyl groups derived from bisphenol A, epoxy resin having glycidyl groups derived from bisphenol F, and epoxy resin having glycidyl groups derived from 1,6-hexanediol from the viewpoint of improvement in toughness, impact strength, adhesion, and chemical resistance of the resulting cured product, and more preferably epoxy resin having glycidyl groups derived from bisphenol A as a main component from the viewpoint of obtaining a coating film having high hardness and from the viewpoint of availability and economic performance.

It should be noted that in this context, the "main component" means that other components may be contained without departing from the gist of the present invention, and means preferably 50 to 100 mass%, more preferably 70 to 100 mass%, further preferably 90 to 100 mass%, of the whole.

It should be noted that the epoxy resin preferably comprises at least one member selected from the group consisting of epoxy resin having glycidyl groups derived from bisphenol A with a biomass content of 20% or more, and epoxy resin having glycidyl groups derived from 1,6-hexanediol with a biomass content of 100%, and more preferably has, as a main component, at least one member selected from the group consisting of epoxy resin having glycidyl groups derived from bisphenol A with a biomass content of 20% or more, and epoxy resin having glycidyl groups derived from 1,6-hexanediol with a biomass content of 100%, from the viewpoint of improving the biomass content of the epoxy resin composition.

The content ratio between the epoxy resin and the epoxy resin curing agent in the epoxy resin composition of the present invention is an amount such that the ratio of the number of active hydrogen atoms in the epoxy resin curing agent to the number of epoxy groups in the epoxy resin (the number of active hydrogen atoms in the epoxy resin curing agent/the number of epoxy groups in the epoxy resin) is preferably 1/0.5 to 1/2, more preferably 1/0.75 to 1/1.5, further preferably 1/0.8 to 1/1.2.

The content of the main agent epoxy resin in the epoxy resin composition of the present invention is preferably 30 to 90 mass%, more preferably 40 to 80 mass%, further preferably 40 to 75 mass%, from the viewpoint of improvement in toughness, impact strength, adhesion, and chemical resistance of the resulting cured product.

Furthermore, the content of the epoxy resin curing agent in the epoxy resin composition of the present invention is preferably 10 to 70 mass%, more preferably 15 to 50 mass%, further preferably 15 to 40 mass%, from the viewpoint of improvement in toughness, impact strength, adhesion, and chemical resistance of the resulting cured product.

Furthermore, the epoxy resin composition of the present invention may further comprise at least one additive selected from the group consisting of a coupling agent, a flame retardant, an antioxidant, a light stabilizer, a plasticizer, a diluent, a curing accelerator, a pigment, an inorganic filler, and an organic filler without impairing the advantageous effects of the present invention, and preferably comprises an additive suitable for the intended use. Among them, a curing accelerator is preferably contained therein.

Examples of the curing accelerator include phenolic compounds, organic acids, organic acid salts, tertiary amines, quaternary ammonium salts, imidazoles, organic phosphorus compounds, quaternary phosphonium salts, diazabicycloalkenes, organic metal salt compounds, boron compounds, and metal halides.

However, the total content of the epoxy resin and the epoxy resin curing agent in the epoxy resin composition is preferably 50 mass% or more, more preferably 70 mass% or more, further preferably 80 mass% or more, and 100 mass% or less, from the viewpoint of effectively obtaining the advantageous effects of the present invention.

Furthermore, the epoxy resin composition of the present invention may contain a solvent (water and volatile organic solvents) other than the diluent, and its content in the epoxy resin composition is preferably 5 mass% or less, more preferably 2 mass% or less, further preferably 1 mass% or less.

It should be noted that the amine compound (a) and the modified product (A) thereof have a low viscosity and therefore produce a cured product having favorable properties substantially without the use of the diluent, when used as an epoxy resin curing agent contained in the epoxy resin composition. Thus, the epoxy resin composition of the present invention preferably comprises no diluent or comprises a diluent in the content of less than 50 mass%. Such a decreased amount of the diluent used can elevate the concentration of the amine compound (a) produced using a biomass-derived starting material in the epoxy resin composition and by extension, can improve the biomass content of an epoxy resin composition.

On the other hand, polyaminoamide-based curing agents heretofore used have a high viscosity and therefore require a diluent and have the difficulty in improving the biomass content of an epoxy resin composition.

### <Method for producing epoxy resin composition>

A method for producing the epoxy resin composition is not particularly limited, and the epoxy resin composition can be produced by mixing the epoxy resin, the epoxy resin curing agent, and other optional components by a known method using a known apparatus. The order of mixing the individual components contained in the epoxy resin composition is not particularly limited either. The epoxy resin curing agent may be prepared and then mixed with the epoxy resin, or the amine compound (a) or the modified product (A) thereof and other components such as additives constituting the epoxy resin curing agent, and the epoxy resin may be mixed at the same time for production.

### [Paint]

The present invention provides a paint comprising the epoxy resin composition. The paint of the present invention comprises the epoxy resin composition and thereby quickly cures and is capable of forming a coating film excellent in toughness, impact strength, adhesion, and chemical resistance. Examples of the paint include paints such as marine paints, heavy-duty corrosion-resistant paints, paints for tanks, pipe interior coating paints, exterior paints, and floor paints.

The content of the epoxy resin composition in the paint of the present invention is preferably 50 mass% or more, more preferably 70 mass% or more, further preferably 80 mass% or more, still further preferably 90 mass% or more, still further preferably 95 mass% or more, and 100 mass% or less, from the viewpoint of improvement in toughness, impact strength, adhesion, and chemical resistance of the resulting cured product.

### [Cured product]

A cured product of the epoxy resin composition of the present invention (hereinafter, also simply referred to as the "cured product of the present invention") is a cured product obtained by curing the epoxy resin composition, and is obtained by curing the epoxy resin composition by a known method. The curing conditions for the epoxy resin composition are not particularly limited and are appropriately selected depending on the intended use and form of the resulting cured product.

The form of the cured product of the present invention is not particularly limited either and can be selected depending on the intended use. The cured product of the epoxy resin composition is preferably a cured product in the form of a film from the viewpoint of forming a coating film.

Furthermore, the cured product of the present invention may be a molded article.

The coating film which is the cured product of the epoxy resin composition of the present invention quickly cures and is excellent in toughness, impact strength, adhesion, and chemical resistance.

The biomass content of the cured product of the present invention is preferably 40 mass% or more. The upper limit is not limited and is 100 mass% or less, still further preferably 100 mass%. The biomass content can be adjusted to the above range by using the epoxy resin and the curing agent having a high biomass content in the epoxy resin composition serving as a starting material for the cured product. It should be noted that the biomass content is the proportion of carbon of a biomass-derived component among all carbon atoms in the cured product.

The epoxy resin composition of the present invention has fast curing and can further form a coating film having favorable adhesion and impact resistance, and as such, is suitably used for, for example, paints such as marine paints, heavy-duty corrosion-resistant paints, paints for tanks, pipe interior coating paints, exterior paints, and floor paints.

### <Molded article>

The molded article of the present invention is obtained by molding and curing the epoxy resin composition. The molded article of the present invention can be obtained by a general molding method, for example, casting molding, potting molding, transfer molding, RTM molding, or VARTM molding.

The coating film which is the cured product of the epoxy resin composition of the present invention is excellent in viscosity, compression strength, and compression modulus of elasticity and moreover, also excellent in biomass content.

### [Amine compound]

The amine compound of the present invention is represented by the general formula (1a) given below. Specifically, the amine compound represented by the general formula (1a) given below is also included in the present invention.

The amine compound represented by the formula (1a) is a novel amine compound that can be produced using a starting material derived from biomass among the amine compounds (a) represented by the general formula (1). This amine compound, when used in an epoxy resin curing agent or the like, can produce an epoxy resin curing agent that can produce a cured product excellent in toughness, impact strength, adhesion, and chemical resistance. 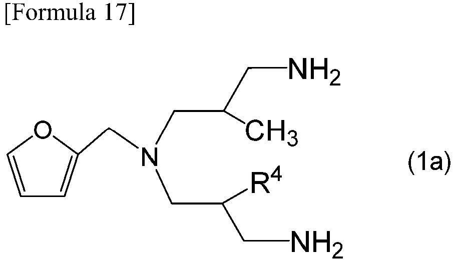 wherein, in the formula (1a), R⁴ is a hydrogen atom or a methyl group.

In the general formula (1a), R⁴ is a hydrogen atom or a methyl group, preferably a hydrogen atom.

It should be noted that the amine compound represented by the general formula (1a) of the present invention may be produced by any method and is preferably produced by the method described in the above section (Method for producing amine compound (a)). Specifically, the method for producing the amine compound represented by the general formula (1a) is a production method comprising the step of hydrogenating a nitrile compound represented by the general formula (2) to give an amine compound represented by the general formula (1a). However, in the formula (2), R¹ is a methylene group, R² is a 2-methylethylene group, and R³ is an ethylene group or a 2-methylethylene group.

### <Intended use of amine compound>

The amine compound of the present invention represented by the formula (1a) can be used for various purposes. Furthermore, the amine compound, when used as a starting material for a resin, is considered to be able to impart toughness, impact strength, adhesion, and chemical resistance to the resulting resin.

The amine compound of the present invention represented by the formula (1a) can be used as, for example, a starting material for polyamide, a starting material for polyimide, a starting material for polyurethane, a starting material for polyurea, and a ligand. Next, each intended use will be described in detail.

### (Intended use as starting material for polyamide)

The amine compound can be suitably used as a starting material for polyamide. Furthermore, the amine compound is considered to be able to impart toughness, impact strength, adhesion, and chemical resistance to the resulting polyamide.

The polyamide can be obtained by introducing the amine compound and a dicarboxylic acid to a reaction system, and performing polycondensation reaction.

The dicarboxylic acid is not particularly limited and is preferably at least one member selected from the group consisting of aliphatic dicarboxylic acid having 4 to 20 carbon atoms, terephthalic acid, and isophthalic acid, more preferably aliphatic dicarboxylic acid having 4 to 20 carbon atoms, further preferably aliphatic dicarboxylic acid having 4 to 12 carbon atoms.

Examples of the aliphatic dicarboxylic acid having 4 to 20 carbon atoms can include succinic acid, glutaric acid, adipic acid, pimelic acid, suberic acid, azelaic acid, sebacic acid, 1,10-decanedicarboxylic acid, 1,11-undecanedicarboxylic acid, 1,12-dodecanedicarboxylic acid, 1,14-tetradecanedicarboxylic acid, 1,16-hexadecanedicarboxylic acid, and 1,18-octadecanedicarboxylic acid, among which at least one member selected from the group consisting of adipic acid and sebacic acid is preferably used from the viewpoint of crystallizability and high elasticity. One of or two or more in combination of these dicarboxylic acids may be used.

Other examples of the dicarboxylic acid include aliphatic dicarboxylic acids having 3 or less carbon atoms, such as oxalic acid and malonic acid; and aromatic dicarboxylic acids other than terephthalic acid and isophthalic acid, such as 2,6-naphthalenedicarboxylic acid.

The molar ratio between the amine compound and the dicarboxylic acid (amine compound/dicarboxylic acid) is preferably in the range of 0.9 to 1.1, more preferably in the range of 0.93 to 1.07, further preferably in the range of 0.95 to 1.05, further preferably in the range of 0.97 to 1.02. Provided that the molar ratio falls within the above range, increase in molecular weight easily progresses.

### (Intended use as starting material for polyimide)

The amine compound can be suitably used as a starting material for polyimide. Furthermore, the amine compound is considered to be able to impart toughness, impact strength, adhesion, and chemical resistance to the resulting polyimide.

The polyimide can be obtained by introducing the amine compound and a tetracarboxylic acid dianhydride to a reaction system, obtaining polyamic acid through ring-opening polyaddition reaction, and further performing imidization. The ring-opening polyaddition reaction and the imidization may be performed at once.

Examples of the tetracarboxylic acid dianhydride include, but are not particularly limited to, aromatic tetracarboxylic acid dianhydrides, alicyclic tetracarboxylic acid dianhydrides, and aliphatic tetracarboxylic acid dianhydrides.

Examples of the aromatic tetracarboxylic acid dianhydride include biphenyl tetracarboxylic acid dianhydride (BPDA), 9,9-bis(3,4-dicarboxyphenyl)fluorene dianhydride (BPAF), pyromellitic acid dianhydride, 3,3',4,4'-(hexafluoroisopropylidene)diphthalic acid anhydride, 3,3',4,4'-diphenylsulfonetetracarboxylic acid dianhydride, 3,3',4,4'-benzophenonetetracarboxylic acid dianhydride, and 2,2',3,3'-benzophenonetetracarboxylic acid dianhydride.

Examples of the alicyclic tetracarboxylic acid dianhydride that provides a constitutional unit derived from the alicyclic tetracarboxylic acid dianhydride include 1,2,4,5-cyclohexanetetracarboxylic acid dianhydride, 1,2,3,4-cyclobutanetetracarboxylic acid dianhydride, norbornane-2-spiro-α-cyclopentanone-α'-spiro-2"-norbornane-5,5",6,6"-tetracarboxylic acid dianhydride, bicyclo[2.2.2]oct-7-ene-2,3,5,6-tetracarboxylic acid dianhydride, dicyclohexyltetracarboxylic acid dianhydride, 5,5'-bis-2-norbornene-5,5',6,6'-tetracarboxylic acid-5,5',6,6'-dianhydride, and their positional isomers.

Examples of the aliphatic tetracarboxylic acid dianhydride that provides a constitutional unit derived from the aliphatic tetracarboxylic acid dianhydride include 1,2,3,4-butanetetracarboxylic acid dianhydride. One of or two or more in combination of these tetracarboxylic acid dianhydrides may be used.

### <Intended use as starting material for polyurethane>

The amine compound can be suitably used as a starting material for polyurethane. Furthermore, the amine compound is considered to be able to impart toughness, impact strength, adhesion, and chemical resistance to the resulting polyurethane.

The amine compound is first reacted with phosgene to give corresponding isocyanate. The obtained isocyanate can be reacted with a polyol to give polyurethane.

Examples of the polyol include, but are not particularly limited to, polyester polyols, polyether polyols, polycarbonate polyols, and polylactone polyols.

Examples of the polyester polyol include condensed polyester polyols such as polyethylene adipate glycol, polybutylene adipate glycol, polyhexamethylene adipate glycol, and polyethylene butylene adipate glycol.

Examples of the polyether polyol include aliphatic polyether polyols such as polytetramethylene glycol, polyethylene glycol, and polypropylene glycol.

Examples of the polycarbonate polyol include polyols obtained through the dealcoholization reaction of a low-molecular polyol such as ethylene glycol, propylene glycol, butanediol, pentanediol, hexanediol, octanediol, nonanediol, or 1,4-cyclohexanedimethanol, and a carbonate compound such as diethylene carbonate, dipropylene carbonate, or diphenyl carbonate.

Examples of the polylactone polyol include polylactone diol obtained by the ring-opening polymerization of lactone using an initiator such as the above low-molecular polyol, and lactone polyester diol such as polycaprolactone diol and polymethylvalerolactone diol.

One of these polyols can be used singly, or two or more thereof can be used in combination.

### <Intended use as starting material for polyurea>

The amine compound can be suitably used as a starting material for polyurea. Furthermore, the amine compound is considered to be able to impart toughness, impact strength, adhesion, and chemical resistance to the resulting polyurea.

The amine compound can be reacted with a polyisocyanate to give polyurea.

The polyisocyanate is not particularly limited as long as the polyisocyanate contains a compound having two or more isocyanate groups. The polyisocyanate is preferably diisocyanate having two or more isocyanate groups.

Examples of the diisocyanate having two isocyanate groups include aliphatic isocyanate compounds such as 1,6-hexamethylene diisocyanate (HDI), 2,2,4-trimethylhexamethylene diisocyanate, methylene diisocyanate, isopropylene diisocyanate, lysine diisocyanate, lysine diisocyanate methyl ester, and 1,5-octylene diisocyanate; alicyclic isocyanate compounds such as 4,4'-dicyclohexylmethane diisocyanate, isophorone diisocyanate (IPDI), norbornene diisocyanate, hydrogenated tolylene diisocyanate, methylcyclohexane diisocyanate, isopropylidene bis(4-cyclohexyl isocyanate), and dimer acid diisocyanate; and aromatic isocyanate compounds such as 2,4- or 2,6-tolylene diisocyanate (TDI), 4,4'-diphenylmethane diisocyanate (MDI), 1,5-naphthylene diisocyanate, p- or m-xylylene diisocyanate (XDI), tolidine diisocyanate, p-phenylene diisocyanate, diphenyl ether diisocyanate, diphenylsulfone diisocyanate, dianisidine diisocyanate, and tetramethyl-m-xylylene diisocyanate.

Examples of the polyisocyanate having three or more isocyanate groups include triphenylmethane triisocyanate, triisocyanatophenyl thiophosphate, polymethylene polyphenylene polyisocyanate (polymeric MDI), modified products of isocyanurate which are HDI or TDI trimers, and modified products of biuret.

One of these polyisocyanates can be used singly, or two or more thereof can be used in combination.

### <Intended use as ligand>

The amine compound can be suitably used as a ligand. The amine compound is considered to be able to be used as a ligand in a metal catalyst or the like. Furthermore, the amine compound is considered to be able to be used as a chelating agent for the removal or separation of a metal ion.

The above metal or the metal for use as the metal ion is preferably an element of groups 3 to 11, more preferably a transition metal, further preferably a noble metal.

### <Other intended uses>

The amine compound of the present invention, and the above polyamide resin, polyimide resin, polyurethane resin, polyurea resin, and the like obtained using the amine compound of the present invention can be used in various purposes typified by, for example, various molded articles, various chassis, paints, adhesives, composite materials, insulating materials, sealing materials, molding powders, liners, various coatings, water treatment agents, fiber treatment agents, dispersing agents, surfactants, bleaching agents, corrosion inhibitors, paper strengthening agents, chain extenders, connecters, CO₂ absorbents, dielectric materials, various films, electronic substrate materials, fiber materials, and switches.

However, the intended use of the amine compound of the present invention is not limited to the above intended uses or purposes.

### Examples

The present invention will be described below in detail with reference to examples and comparative examples. However, the present invention is not limited to the examples described below.

### [Analysis of amine compound and epoxy resin curing agent]

### (Gas chromatography analysis conditions)

Equipment used: Gas chromatography 8860GC (manufactured by Agilent Technologies, Inc.)
Column: DB-1 (length: 30 m, inner diameter: 530 µm, film thickness: 1.5 µm)
Detector: FID (H₂: 40 mL/min, air: 450 mL/min)
Carrier gas: He
Split ratio: 5:1
Injection port temperature: 250°C
Detector temperature: 250°C
Injection volume: 1.0 µL
Oven temperature: held at 60°C for 6 minutes, raised from 60°C to 280°C at 7°C/min, and held for 20 minutes after reaching 280°C.

### (GC-MS)

### (GC)

Equipment used: Gas chromatography GC2010 Plus (manufactured by Shimadzu Corp.)
Column: DB-1MS (length: 30 m, inner diameter: 0.25 mm, film thickness: 0.25 µm)
Carrier gas: He
Split ratio: 5:1
Vaporizing chamber temperature: 250°C
Injection volume: 1 µL
Oven temperature: held at 60°C for 6 minutes, raised from 60°C to 280°C at 7°C/min, and held for 20 minutes after reaching 280°C.

### (MS)

Equipment used: GCMS-QP2010 Ultra (manufactured by Shimadzu Corp.)
Fragmentation method: EI
Ion source temperature: 200°C
Interface temperature: 250°C

### (Total amine value)

The total amine value of an amine compound was measured by the potentiometric titration method in accordance with JIS K 7237: 1995 using an automatic potentiometric titrator "AT-710S" manufactured by Kyoto Electronics Manufacturing Co., Ltd. However, the measurement was carried out by changing a sample solvent from the o-nitrotoluene/acetic acid solution described in JIS to acetic acid.

### (Active hydrogen equivalent weight (AHEW))

AHEW of an amine compound and an epoxy resin curing agent was obtained by determining a total amine value using an automatic potentiometric titrator "AT-710S" manufactured by Kyoto Electronics Manufacturing Co., Ltd., and calculating AHEW from the results. The total amine value was measured using a 0.1 mol/L solution of perchloric acid in acetic acid (manufactured by Kanto Chemical Co., Inc.).

The structural identification of an amine compound obtained in an example was performed by NMR analysis under the following conditions.

### (NMR analysis method)

Equipment used: AVANCE NEO model 400 NMR apparatus manufactured by Bruker Japan K.K.
Resonance frequency: 400 MHz
Mode: proton (¹H), carbon (¹³C)
Measurement temperature: room temperature
Solvent: CDCl₃ (deuterated chloroform)
Chemical shift reference substance: tetramethylsilane

### [Evaluation of epoxy resin composition and cured product]

### (Dryness (evaluation of curing rate))

The base material used was a zinc phosphate-treated steel plate (manufactured by PALTEK CORPORATION; SPCC-SDPB-N144 0.8 mm **×** 70 mm **×** 150 mm). The base material was coated with an epoxy resin composition of each example using an applicator under conditions of 23**°**C and 50% R.H. to form a coating film (coating film thickness just after coating: 200 **µ**m). This coating film was stored under conditions of 23**°**C and 50% R.H. and evaluated by finger touch after a lapse of 1, 2, and 7 days according to the criteria given below. The results are shown in Table 1.

Ex: Excellent (Even when the coating film is pressed with a thumb at a force of about 50 N, there is no stickiness and no fingerprint is left.)

G: Good (Even when the coating film is pressed with a thumb at a force of about 50 N, there is no stickiness, but a fingerprint is left after finger touch.)

F: Fair (When the coating film is pressed with a thumb at a force of about 50 N, there is stickiness).

P: Poor (When the coating film is pressed with a thumb at a force of about 5 N, there is stickiness)

### (Erichsen test (evaluation of toughness))

In the same manner as described above, a base material (zinc phosphate-treated steel plate) was coated with an epoxy resin composition using an applicator to form a coating film (thickness just after coating: 200 µm). This coating film was stored under conditions of 23**°**C and 50% R.H. After a lapse of 7 days, the coating film was subjected to an Erichsen test under conditions of 23**°**C and 50% R.H in accordance with JIS K 5600-5-2: 1999 (cupping resistance) using an Erichsen film strength tester ("HD-4510" manufactured by Ueshima Seisakusho Co., Ltd.) to measure the minimum indentation depth until a defect occurred in the coating film. The results are shown in Table 1. A larger value indicates that the coating film has higher following properties to deformation and has better toughness.

### (Dupont impact test (evaluation of impact strength))

In the same manner as described above, a base material (zinc phosphate-treated steel plate) was coated with an epoxy resin composition to form a coating film (thickness just after coating: 200 µm). This coating film was stored under conditions of 23**°**C and 50% R.H. After a lapse of 7 days, the coating film was subjected to a Dupont impact test (test on resistance to falling weight) in accordance with JIS K 5600-5-3: 1999.

A 500 g weight was dropped from heights of 50 cm, 30 cm, 20 cm, and 10 cm onto each of the coating film surface (front) and the back surface using a Dupont falling impact tester (manufactured by Mys-Shikenki Co., Ltd.), and a height at which the coating film resisted the impact is shown in Table 1. A larger value indicates that the coating film has higher impact resistance. It should be noted that the case in which the coating film did not resist the impact from the height of 10 cm was indicated in "10F".

### (Cross-cut peeling test (evaluation of adhesion))

A cross-cut was made by a method abiding by JIS K 5600-5-6: 1999, and a coating film was evaluated for its adhesion with a substrate. In the same manner as described above, a base material (zinc phosphate-treated steel plate) was coated with an epoxy resin composition to form a coating film (thickness just after coating: 200 µm). This coating film was stored under conditions of 23**°**C and 50% R.H. After a lapse of 7 days, six incisions reaching the base material were made in the coating film surface using a cutter knife. In this respect, the gap between the incisions was set to 1 mm. In the same manner as above, six incisions reaching the base material were made from above the incisions in a direction orthogonal to the incisions to create 25 grids of 1 mm square. Scotch tape was strongly pressure-bonded to the grid portion. The edge of the tape was peeled at once with an angle of 45**°**, and the number of grids remaining on the base material was visually confirmed. The number X of grids remaining on the base material is shown as "X/25" in Table 1. A larger value of X indicates that the coating film is more peel-resistant and has better adhesion with the base material.

### (Test on chemical resistance of coating film)

In the same manner as in the test on dryness (evaluation of curing rate), a base material (zinc phosphate-treated steel plate) was coated with an epoxy resin composition to form a coating film (thickness just after coating: 200 µm). This steel plate with the coating film formed thereon was dipped in testing chemical solutions given below, and left at 23**°**C. After a lapse of 1, 2, 3, and 4 weeks from the dipping, change in appearance was visually observed, and chemical resistance was thereby evaluated according to the criteria given below. The results are shown in Table 2.

### (Testing chemical solution)

10 mass% aqueous sodium hydroxide solution, aqueous methanol, toluene, and pure water

### (Evaluation criteria)

Ex: Excellent (There is no change.)
G: Good (Initial appearance is substantially maintained.)
F: Fair (Appearance is slightly changed.)
P: Poor (Appearance is changed.)

### (Salt spray test)

A zinc phosphate-treated steel plate (manufactured by PALTEK CORPORATION; SPCC-SDPB-N144 0.8 **×** 70 **×** 150 mm) was coated with an epoxy resin composition of each example using an applicator to form a coating film (thickness just after coating: 200 µm). Subsequently, a uncoated portion of the zinc phosphate-treated steel plate was sealed with rust-proof paints ("MILLION PRIMER" and "MILLION CLEAR" manufactured by Kansai Paint Co., Ltd.) and stored for 7 days under conditions of 23**°**C and 50% R.H for curing to prepare a test specimen. Subsequently, incisions reaching the base material were made using a cutter knife having a blade thickness of 0.38 mm. The incisions were 43 cm each of two straight lines made in an X-shaped pattern, and an angle formed by the two incisions was set to 60 degrees. A 5% salt spray test was conducted by the following method using the test specimen.

The above test specimen was loaded in a salt spray tester ("STP-90" manufactured by Suga Test Instruments Co., Ltd., chamber temperature: 35°C), and salt water (concentration: 5 mass%) was continuously sprayed thereto. After a lapse of 1, 2, 3, and 4 weeks, appearance (overall and cut portion) was visually observed and evaluated according to the criteria given below. The presence or absence of occurrence of a rust spot was confirmed by visually observing the base material (zinc phosphate-treated steel plate) surface in contact with the coating film. Furthermore, the incised portion was also evaluated for a "rust width" described below.

In Table 2, the "overall appearance" is evaluation results obtained by the visual observation of the whole coating film, and the "cut portion" is evaluation results obtained by the visual observation of the X-shaped incisions and their neighborhood.

Ex: No rust spot is confirmed on the coating film surface, and there is no change in coating film appearance.

G: The occurrence of a rust spot in a small amount can be confirmed on the coating film surface, but there is no problem for use, or the gloss of the coating film is reduced.

F: The occurrence of a rust spot can be confirmed on the coating film surface, or the gloss of the coating film is significantly reduced.

P: The occurrence of a rust spot in a large amount can be confirmed on the coating film surface, or blister occurs.

In Table 2, the "rust width" is a horizontal width (mm) of rust developed and deteriorated from the X-shaped incisions, and a smaller value of the "rust width" is more preferred because of better adhesion of the coating film.

### [Production of amine compound (a)]

### Production Example 1 (production of 3-amino-1-[(3-aminopropyl)(furfuryl)amino]propane (AFDA))

### (1) Unsaturated nitrile (acrylonitrile) addition reaction step

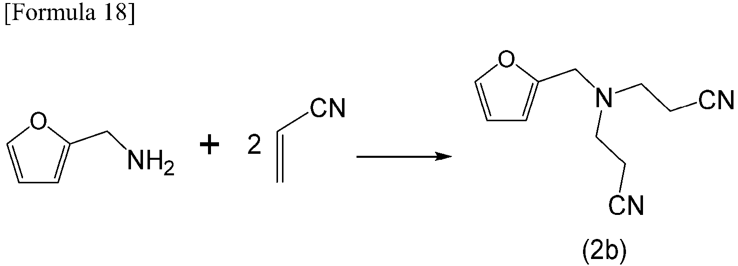

A flask equipped with a condenser was charged with 30 g (0.31 mol) of furfurylamine, 66 g (1.24 mol) of acrylonitrile, and 100 g of water and heated into total reflux with stirring, followed by reaction for 30 hours.

After cooling, acrylonitrile was removed with an evaporator to give 66 g of a concentrate containing a nitrile compound represented by the formula (2b).

### (2) Nitrile hydrogenation step

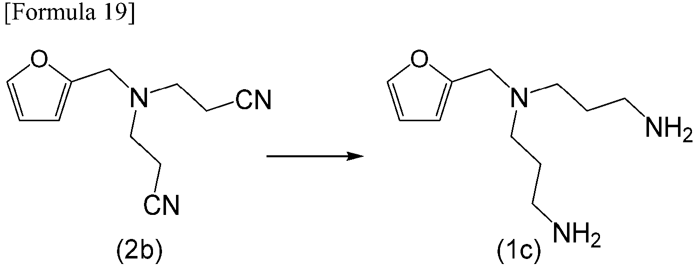

Next, an autoclave was charged with 10 g of the concentrate, 1.00 g of a sponge cobalt catalyst (RANEY(registered trademark) 2724, manufactured by W.R. Grace & Co.) (moisture content: 40.0%, dry mass: 0.60 g), and 70 g of tetrahydrofuran. After the reactor was purged with nitrogen and hydrogen, hydrogen was introduced thereto so as to attain a hydrogen pressure of 8.0 MPa, and reaction was performed at a reaction temperature of 120°C for 5 hours with stirring. After subsequent cooling, impurities were removed by simple distillation to give an amine compound represented by the formula (1c) (3-amino-1-[(3-aminopropyl)(furfuryl)amino]propane (AFDA)).

Results of analyzing the obtained 3-amino-1-[(3-aminopropyl)(furfuryl)amino]propane (AFDA) by NMR are shown. 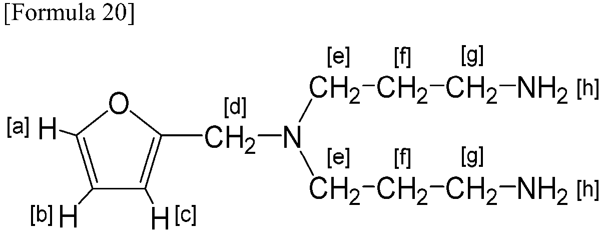 ¹H-NMR chemical shift: δ: 1.10 (s, 4H, [h]), 1.59-1.66 (m, 4H, [f]), 2.47-2.50 (t, 4H, [e]), 2.70-2.74 (t, 4H, [g]), 3.63 (s, 2H, [d]), 6.16-6.17 (m, 1H, [c]), 6.30-6.31 (m, 1H, [b]), 7.35-7.36 (m, 1H, [a]) 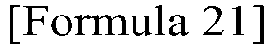 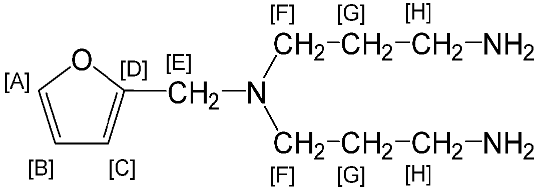 ¹³C-NMR chemical shift: δ: 31.09, 40.59, 50.00, 51.55, 108.22, 109.97, 141.75, 152.77
31.09 is derived from the carbon atom of [G]. 40.59 is derived from the carbon atom of [H]. 50.00 is derived from the carbon atom of [E]. 51.55 is derived from the carbon atom of [F]. 108.22 is derived from the carbon atom of [C]. 109.97 is derived from the carbon atom of [B]. 141.75 is derived from the carbon atom of [A]. 152.77 is derived from the carbon atom of [D].

Furthermore, results of analyzing the obtained 3-amino-1-[(3-aminopropyl)(furfuryl)amino]propane (AFDA) by GC-MS showed 211 based on the molecular weight 211 of AFDA.

### [Epoxy resin curing agent and epoxy resin composition]

### Example 1

### (Production of epoxy resin curing agent containing AFDA)

The 3-amino-1-[(3-aminopropyl)(furfuryl)amino]propane (AFDA) was diluted with benzyl alcohol added as a non-reactive diluent in an amount of 40 mass% of the whole to give an epoxy resin curing agent having a concentration of the AFDA of 60 mass%. The amino group-derived active hydrogen equivalent weight (AHEW) of the epoxy resin curing agent (whole amount including benzyl alcohol) was 88.0 g/equivalent weight.

### (Production of epoxy resin composition)

The main agent epoxy resin used was liquid epoxy resin having glycidyl groups derived from bisphenol A ("jER828" manufactured by Mitsubishi Chemical Corp., epoxy equivalent weight: 186 g/equivalent weight).

The epoxy resin and the epoxy resin curing agent were blended according to the formulation shown in Table 1 and mixed with stirring at 23°C to prepare an epoxy resin composition. The ratio of the number of active hydrogen atoms in the epoxy resin curing agent to the number of epoxy groups in the main agent epoxy resin (the number of active hydrogen atoms in the epoxy resin curing agent/the number of epoxy groups in the main agent epoxy resin) was set to 1/1.

Evaluation was performed by the above methods using the obtained epoxy resin composition. The results are shown in Table 1 and Table 2.

### Comparative Example 1

### (Production of epoxy resin curing agent and epoxy resin composition containing diethylenetriamine (DETA))

An epoxy resin curing agent and an epoxy resin composition were produced in the same manner as in Example 1 except that diethylenetriamine (DETA) was used instead of AFDA in Example 1. Evaluation was performed by the above methods using the obtained epoxy resin composition. The results are shown in Table 1.

### Example 2

### (Production of epoxy resin curing agent containing epoxy modified product (AFDA 5/1 adduct) of AFDA)

A separable flask having an internal capacity of 300 milliliters and equipped with a stirrer, a thermometer, a nitrogen inlet tube, a dropping funnel, and a condenser was charged with 29.6 g of the AFDA obtained in Example 1, and polyfunctional epoxy resin having glycidyl groups derived from bisphenol A ("jER828" manufactured by Mitsubishi Chemical Corp., epoxy equivalent weight: 186 g/eq.) was added dropwise thereto as an epoxy compound in an amount of 10.4 g (corresponding to an amount such that the number of active hydrogen atoms in the AFDA/the number of epoxy groups in the epoxy compound = 10/1) over 2 hours in a nitrogen stream with stirring. After the completion of the dropwise addition, the temperature was raised to 80**°**C, and reaction was performed for 2 hours to give a reaction product of the epoxy compound and the AFDA (AFDA 5/1 adduct; AFDA/epoxy compound adduct ratio: 5/1). The reaction product was diluted with benzyl alcohol added as a non-reactive diluent in an amount of 40 mass% of the whole to give an epoxy resin curing agent having a concentration of 60 mass%. The active hydrogen equivalent weight (AHEW) of the epoxy resin curing agent (whole amount including benzyl alcohol) was 132.3 g/equivalent weight.

### (Production of epoxy resin composition)

The main agent epoxy resin used was liquid epoxy resin having glycidyl groups derived from bisphenol A ("jER828" manufactured by Mitsubishi Chemical Corp., epoxy equivalent weight: 186 g/equivalent weight).

The epoxy resin and the epoxy resin curing agent were blended according to the formulation shown in Table 1 and mixed with stirring at 23°C to prepare an epoxy resin composition. The ratio of the number of active hydrogen atoms in the epoxy resin curing agent to the number of epoxy groups in the main agent epoxy resin (the number of active hydrogen atoms in the epoxy resin curing agent/the number of epoxy groups in the main agent epoxy resin) was set to 1/1.

Evaluation was performed by the above methods using the obtained epoxy resin composition. The results are shown in Table 1 and Table 2.

### Comparative Example 2

### (Production of epoxy resin curing agent and epoxy resin composition containing epoxy modified product of diethylenetriamine (DETA) (DETA 5/1 adduct))

An epoxy resin curing agent and an epoxy resin composition were produced in the same manner as in Example 2 except that diethylenetriamine (DETA) was used instead of AFDA in Example 2. Evaluation was performed by the above methods using the obtained epoxy resin composition. The results are shown in Table 1.

### Example 3

### (Production of epoxy resin curing agent containing epoxy modified product of AFDA (AFDA 2/1 adduct))

A separable flask having an internal capacity of 300 milliliters and equipped with a stirrer, a thermometer, a nitrogen inlet tube, a dropping funnel, and a condenser was charged with 11.7 g of the AFDA obtained in Example 1, and polyfunctional epoxy resin having glycidyl groups derived from bisphenol A ("jER828" manufactured by Mitsubishi Chemical Corp., epoxy equivalent weight: 186 g/eq.) was added dropwise thereto as an epoxy compound in an amount of 10.3 g (corresponding to an amount such that the number of active hydrogen atoms in the AFDA/the number of epoxy groups in the epoxy compound = 4/1) over 2 hours in a nitrogen stream with stirring. After the completion of the dropwise addition, the temperature was raised to 80**°**C, and reaction was performed for 2 hours to give a reaction product of the epoxy compound and the AFDA. The reaction product was diluted with benzyl alcohol added as a non-reactive diluent in an amount of 40 mass% of the whole to give an epoxy resin curing agent having a concentration of 60 mass%. The active hydrogen equivalent weight (AHEW) of the epoxy resin curing agent (whole amount including benzyl alcohol) was 220.7 g/equivalent weight.

### (Production of epoxy resin composition)

The main agent epoxy resin used was liquid epoxy resin having glycidyl groups derived from bisphenol A ("jER828" manufactured by Mitsubishi Chemical Corp., epoxy equivalent weight: 186 g/equivalent weight).

The epoxy resin and the epoxy resin curing agent were blended according to the formulation shown in Table 1 and mixed with stirring at 23**°**C to prepare an epoxy resin composition. The ratio of the number of active hydrogen atoms in the epoxy resin curing agent to the number of epoxy groups in the main agent epoxy resin (the number of active hydrogen atoms in the epoxy resin curing agent/the number of epoxy groups in the main agent epoxy resin) was set to 1/1.

Evaluation was performed by the above methods using the obtained epoxy resin composition. The results are shown in Tables 1 and 2.

### Comparative Example 3

### (Production of epoxy resin curing agent and epoxy resin composition containing epoxy modified product of diethylenetriamine (DETA) (DETA 2/1 adduct))

An epoxy resin curing agent and an epoxy resin composition were produced in the same manner as in Example 3 except that diethylenetriamine (DETA) was used instead of AFDA in Example 3. Evaluation was performed by the above methods using the obtained epoxy resin composition. The results are shown in Tables 1 and 2.

### [Table 1]

**Table 1**

| | | | | Example 1 | Comparative Example 1 | Example 2 | Comparative Example 2 | Example 3 | Comparative Example 3 |
|---|---|---|---|---|---|---|---|---|---|
| Epoxy resin composition | Epoxy resin curing agent | AFDA | (parts by mass) | 60 | - | - | - | - | - |
| | | DETA | | - | 60 | - | - | - | - |
| | | Epoxy modified product of AFDA (AFDA 5/1 adduct*⁾) | | - | - | 60 | - | - | - |
| | | Epoxy modified product of DETA (DETA 5/1 adduct*⁾) | | - | - | - | 60 | - | - |
| | | Epoxy modified product of AFDA (AFDA 2/1 adduct*⁾) | | - | - | - | - | 60 | - |
| | | Epoxy modified product of DETA (DETA 2/1 adduct*⁾) | | - | - | - | - | - | 60 |
| | | Non-reactive diluent (benzyl alcohol) | | 40 | 40 | 40 | 40 | 40 | 40 |
| | | AHEW | (g/eq.) | 88.0 | 34.3 | 132.3 | 82.2 | 220.7 | 120.5 |
| | Main agent | Epoxy resin (jER828) | (parts by mass) | 211 | 542 | 141 | 226 | 84 | 154 |
| Evaluation of epoxy resin composition | | Dryness (evaluation of curing rate) (1d/2d/7d) | | Ex/Ex/Ex | G/G/G | Ex/Ex/Ex | G/G/G | Ex/Ex/Ex | F/G/Ex |
| Evaluation of cured product (coating film) | | Erichsen test (evaluation of toughness) (mm) | | 6.23 | Not measurable due to heavy stickiness | 6.74 | 1.52 | 8.32 | 4.58 |
| | | Dupont impact test (evaluation of impact strength) (front/back) | | 10/10F | | 40/20 | 10F/10F | >50/>50 | 20/10F |
| | | Cross-cut peeling test (evaluation of adhesion) | | 1/25 | | 23/25 | 0/25 | 5/25 | 0/25 |

| | | | | | | | | | |
|---|---|---|---|---|---|---|---|---|---|
| *) 5/1 and 2/1 represent an adduct ratio (amine compound/epoxy compound). | | | | | | | | | |

### [Table 2]

**Table 2**

| | | | | Example 1 | Example 2 | Example 3 | Comparative Example 3 |
|---|---|---|---|---|---|---|---|
| Epoxy resin composition | Epoxy resin curing agent | AFDA | (parts by mass) | 60 | - | - | - |
| | | DETA | | - | - | - | - |
| | | Epoxy modified product of AFDA (AFDA 5/1 adduct*⁾) | | - | 60 | - | - |
| | | Epoxy modified product of DETA (DETA 5/1 adduct*⁾) | | - | - | - | - |
| | | Epoxy modified product of AFDA (AFDA 2/1 adduct*⁾) | | - | - | 60 | - |
| | | Epoxy modified product of DETA (DETA 2/1 adduct*⁾) | | - | - | - | 60 |
| | | Non-reactive diluent (benzyl alcohol) | | 40 | 40 | 40 | 40 |
| | | AHEW | (g/eq.) | 88 | 132.3 | 220.7 | 120.5 |
| | Main agent | Epoxy resin (jER828) | (parts by mass) | 211 | 141 | 84 | 154 |
| Evaluation of cured product (coating film) | Test on chemical resistance of coating film (1/2/3/4 weeks later) | 10 mass% aqueous sodium hydroxide solution | | Ex/G/G/G | Ex/G/G/G | Ex/Ex/Ex/Ex | P/-/-/- |
| | | Aqueous methanol | | Ex/Ex/Ex/Ex | Ex/Ex/Ex/Ex | Ex/Ex/Ex/Ex | P/-/-/- |
| | | Toluene | | Ex/Ex/Ex/Ex | Ex/Ex/Ex/Ex | Ex/Ex/Ex/Ex | Ex/G/G/G |
| | | Pure water | | G/G/G/G | Ex/Ex/Ex/Ex | Ex/Ex/Ex/Ex | F/F/P/P |
| | Salt spray test (1/2/3/4 weeks later) | Overall appearance | | Ex/G/G/F | Ex/G/G/F | Ex/G/G/G | F/P/P/P |
| | | Cut portion | | Ex/Ex/G/F | Ex/Ex/F/F | Ex/Ex/G/P | F/P/P/P |
| | | Rust width | | <1/1/1.5/1.5 | <1/1/1.5/1.5 | <1/<1/1/3 | 1.5/2/2/2 |

| | | | | | | | |
|---|---|---|---|---|---|---|---|
| *) 5/1 and 2/1 represent an adduct ratio (amine compound/epoxy compound). | | | | | | | |

As is evident from the results of Table 1, the cured products obtained by the methods of the examples have a large value obtained by the Erichsen test, also have a large value of the height in the Dupont impact test, have a large number of grids remaining on the base material in the cross-cut peeling test, and provide peel-resistant coating films, as compared with the cured products obtained by the methods of the comparative examples. Furthermore, as is evident from the results of the test on dryness, a curing rate is also excellent.

As is evident from the results of Table 2, the cured products obtained by the methods of the examples have no change even when dipped for a long period in an aqueous sodium hydroxide solution, aqueous methanol, toluene, and water and also undergo less occurrence of rust or deterioration of gloss in the salt spray test, as compared with the cured products obtained by the methods of the comparative examples. Thus, as is evident, the cured products obtained by the methods of the examples are excellent in toughness, impact strength, adhesion, and chemical resistance.

As is evident from the above results, the epoxy resin composition containing the epoxy resin curing agent of the present invention can produce a cured product excellent in toughness, impact strength, adhesion, and chemical resistance. The epoxy resin curing agent and the amine compound of the present invention are useful as starting materials for an epoxy resin composition and a cured product.

Furthermore, the epoxy resin curing agent and the amine compound of the present invention can be produced using amine obtained from biomass-derived furfural as a starting material, and biomass-derived acrylonitrile.

### [Epoxy resin curing agent and epoxy resin composition]

### Example 4

### (Production of epoxy resin composition)

The epoxy resin curing agent used was the 3-amino-1-[(3-aminopropyl)(furfuryl)amino]propane (AFDA).

The main agent epoxy resin used was liquid epoxy resin having glycidyl groups derived from bisphenol A ("jER828" manufactured by Mitsubishi Chemical Corp., epoxy equivalent weight: 186 g/equivalent weight).

The epoxy resin, the diluent, and the epoxy resin curing agent were blended according to the formulation shown in Table 3 and mixed with stirring at 23**°**C to prepare an epoxy resin composition. The ratio of the number of active hydrogen atoms in the epoxy resin curing agent to the number of epoxy groups in the main agent epoxy resin (the number of active hydrogen atoms in the epoxy resin curing agent/the number of epoxy groups in the main agent epoxy resin) was set to 1/1.

### Comparative Examples 4 to 6

### (Production of epoxy resin composition containing polyaminoamide-based curing agent)

A polyaminoamide-based curing agent (TOHMIDE 225-X, manufactured by T&K TOKA Corp.) and benzyl alcohol (diluent) in the amount shown in Table 3 were mixed to give an epoxy resin curing agent.

Epoxy resin compositions were produced in the same manner as in Example 4 except that the epoxy resin curing agent shown in Table 3 was used instead of AFDA in Example 4.

### Comparative Example 7

### (Production of epoxy resin composition containing polyaminoamide-based curing agent)

An epoxy resin composition was produced in the same manner as in Example 4 except that a polyaminoamide-based curing agent (TOHMIDE 225-X, manufactured by T&K TOKA Corp.) was used instead of AFDA in Example 4.

### [Evaluation of curing agent and resin composition]

### (Measurement of viscosity (evaluation of formability))

The viscosities of each curing agent and resin composition were measured by the method described in JIS K 7117: 1999 using an E type viscometer. The measurement temperature was set to 25**°**C.

It should be noted that if the viscosity at 25**°**C of the epoxy resin composition exceeds 1000 mPa·s, a molded article (cured product), the compression strength and compression modulus of elasticity of which can be properly measured, cannot be obtained due to poor fluidity or degassing properties in the evaluation of this example. Thus, the epoxy resin curing agent and the epoxy resin composition have a low viscosity and thereby attain excellent formability.

### (Biomass content of curing agent)

The biomass content of the curing agent is the proportion of carbon of a biomass-derived component among all carbon atoms in the curing agent, and was calculated from the proportion of a biomass starting material among the starting materials.

It should be noted that the biomass contents of the curing agents in Comparative Examples 4 and 5 were calculated from the listed value (biomass ration: 65 to 75%) of the polyaminoamide-based curing agent (TOHMIDE 225-X, manufactured by T&K TOKA Corp.).

### [Evaluation of molded article (cured product)]

### (Production of molded article)

Each of the epoxy resin compositions obtained in Example 4 and Comparative Examples 4 and 5 was injected to between two metal plates adjusted so as to attain a thickness of 4 mm in the resulting molded article, and cured for 7 days under conditions of 23**°**C and 50% R.H to give a 4 mm thick molded article.

It should be noted that, as described above, a molded article (cured product), the compression strength and compression modulus of elasticity of which can be properly measured, was unable to be obtained because the epoxy resin compositions obtained in Comparative Examples 6 and 7 had an exceedingly high viscosity. Furthermore, the molded article obtained using the epoxy resin composition obtained in Comparative Example 4 was fragile and was unable to produce a test specimen for a compression test.

### (Evaluation of compression strength and compression modulus of elasticity)

Five 10 mm × 10 mm × 4 mm test specimens were cut out of each of the molded articles using a machining center. Compression strength was measured by the method described in JIS K 7181: 2011, and the obtained values of the compression strength were averaged to calculate the compression strength of the molded article. A molded article having larger compression strength has higher strength and is thus excellent. The results are shown in Table 3.

Furthermore, a compression modulus of elasticity was calculated by dividing compressive strength up to yield point by compressive strain in a compression curve obtained by the measurement of the above compression strength.

### [Table 3]

**Table 3**

| | | | | Example 4 | Comparative Example 4 | Comparative Example 5 | Comparative Example 6 | Comparative Example 7 |
|---|---|---|---|---|---|---|---|---|
| Epoxy resin composition | Epoxy resin curing agent | AFDA | (parts by mass) | 100 | - | - | - | - |
| | | Polyaminoamide-based curing agent | | - | 14 | 50 | 80 | 100 |
| | | Benzyl alcohol (diluent) | | - | 86 | 50 | 20 | - |
| | | Curing agent viscosity (25°C) | (mPa·s) | 19.0 | 23.9 | - | - | - |
| | | AHEW | (g/eq.) | 52.8 | 805 | 230 | 144 | 115 |
| | Main agent | Epoxy resin (jER828) | (parts by mass) | 352 | 23.1 | 80.9 | 129 | 162 |
| | Viscosity of resin composition (25°C) | | (mPa·s) | 910 | 10 or less | 687 | 6,030 | 17,910 |
| Biomass content of curing agent | | | | 45% | 9.3 to 11% | 33 to 38% | Not moldable due to too high viscosity | |
| Evaluation of cured product (molded article) | | Compression strength | (MPa) | 86 | Not measurable | 17.4 | | |
| | | Compression modulus of elasticity | (MPa) | 1,800 | Not measurable | 42.6 | | |

As is evident from the results of Table 3, the molded article (cured product) obtained by the method of the example is excellent in viscosity, compression strength, and compression modulus of elasticity.

As is also evident, the molded article (cured product) obtained by the method of the example is also excellent in biomass content because the curing agent used as a starting material in the molded article has a high biomass content.

Thus, the molded article (cured product) obtained by molding and curing the epoxy resin composition containing the epoxy resin curing agent of the present invention exhibits excellent properties as described above and as such, can be applied to a wide range of purposes such as casted products, plate materials, and block materials, while reducing environmental load.

## Claims

1. An epoxy resin curing agent comprising at least one member selected from the group consisting of an amine compound (a) represented by the following general formula (1), and modified product (A) of the amine compound (a): 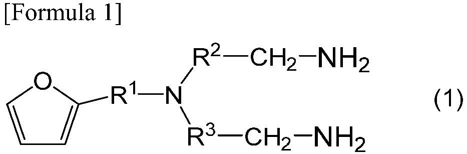 wherein, in the formula (1), R¹ is a linear alkylene group having 1 to 3 carbon atoms, and R² and R³ are each independently a linear or branched alkylene group having 2 to 4 carbon atoms.

2. The epoxy resin curing agent according to claim 1, wherein R¹ is a methylene group, and R² and R³ are each independently an ethylene group or a 2-methylethylene group.

3. The epoxy resin curing agent according to claim 1 or 2, wherein a biomass content is 30 mass% or more.

4. The epoxy resin curing agent according to any one of claims 1 to 3, wherein the modified product (A) of the amine compound (a) comprises at least one member selected from the group consisting of the following (I) to (IV):
(I) a Mannich reaction product of the amine compound (a), a phenolic compound, and an aldehyde compound,
(II) a reaction product of the amine compound (a) and an unsaturated hydrocarbon compound,
(III) a reaction product of the amine compound (a) and an epoxy compound having at least one epoxy group, and
(IV) a reaction product of the amine compound (a) and a carboxylic acid or a derivative thereof.

5. The epoxy resin curing agent according to any one of claims 1 to 4, wherein the modified product (A) of the amine compound (a) is a reaction product of the amine compound (a) and an epoxy compound having at least one epoxy group.

6. The epoxy resin curing agent according to any one of claims 1 to 5, wherein the epoxy resin curing agent comprises no diluent or comprises a diluent in a content of less than 50 mass%.

7. An epoxy resin composition comprising epoxy resin and the epoxy resin curing agent according to any one of claims 1 to 6.

8. The epoxy resin composition according to claim 7, further comprising at least one additive selected from the group consisting of a coupling agent, a flame retardant, an antioxidant, a light stabilizer, a plasticizer, a diluent, a curing accelerator, a pigment, an inorganic filler, and an organic filler.

9. The epoxy resin composition according to claim 7 or 8, wherein the epoxy resin comprises at least one member selected from the group consisting of epoxy resin having glycidyl groups derived from bisphenol A with a biomass content of 20% or more, and epoxy resin having glycidyl groups derived from 1,6-hexanediol with a biomass content of 100%.

10. The epoxy resin composition according to any one of claims 7 to 9, wherein a biomass content is 40 mass% or more.

11. The epoxy resin composition according to any one of claims 7 to 10, wherein the epoxy resin composition comprises no diluent or comprises a diluent in a content of less than 50 mass%.

12. A paint comprising the epoxy resin composition according to any one of claims 7 to 11.

13. A cured product obtained by curing the epoxy resin composition according to any one of claims 7 to 11.

14. The cured product according to claim 13, wherein a biomass content is 40 mass% or more.

15. The cured product according to claim 13 or 14, wherein a compression strength is 70 MPa or more.

16. The cured product according to any one of claims 13 to 15, wherein a compression modulus of elasticity is 1700 MPa or more.

17. The cured product according to any one of claims 13 to 16, wherein the cured product is a molded article.

18. A method for producing an epoxy resin curing agent, comprising the step of hydrogenating a nitrile compound represented by the following general formula (2) to give amine compound (a) represented by the following general formula (1): 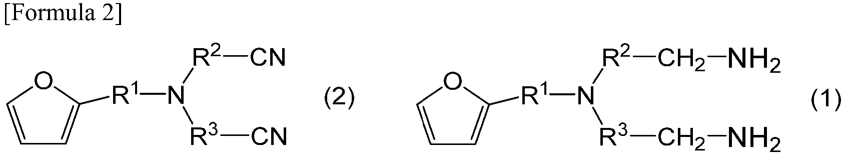 wherein, in the formula (1), R¹ is a linear alkylene group having 1 to 3 carbon atoms, and R² and R³ are each independently a linear or branched alkylene group having 2 to 4 carbon atoms, and in the formula (2), R¹ is a linear alkylene group having 1 to 3 carbon atoms, and R² and R³ are each independently a linear or branched alkylene group having 2 to 4 carbon atoms.

19. An amine compound represented by the following general formula (1a): 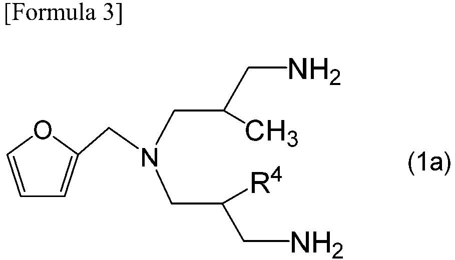 wherein, in the formula (1a), R⁴ is a hydrogen atom or a methyl group.
